# EUROPEAN PATENT APPLICATION

(11) **EP 3 950 699 A1**
(43) Date of publication of application: **09.02.2022**
(21) Application number: 20784783.1
(22) Date of filing: 02.04.2020
(51) Int. Cl.: C07H 21/02, C12P 19/34, C12N 15/11

(54) **RNA CAPPING METHOD, PRODUCTION METHOD FOR MODIFIED RNA, AND MODIFIED RNA**

(30) Priority: 05.04.2019 JP 2019073163
(71) Applicant: Kyoto University, Kyoto-shi, Kyoto 606-8501 (JP)
(72) Inventor: SAITO Hirohide, Kyoto-shi, Kyoto 606-8501 (JP); OHNO Hirohisa, Kyoto-shi, Kyoto 606-8501 (JP); AKAMINE Sae, Kyoto-shi, Kyoto 606-8501 (JP)
(74) Representative: Cabinet Becker et Associés
(86) International application number: PCT/JP2020/015168
(87) International publication number: WO 2020/204130

(57) **Abstract**

An RNA capping method including a step of reacting a compound (1) represented by the following general formula (1) with an RNA in the presence of a Vaccinia virus capping enzyme (R^{b1} represents an oxo group, an alkoxy group, or a halogen atom; R^{b2} is either absent or represents an alkyl group; R^{b3} represents an amino group or a hydrogen atom; R^{b4} is either absent or represents a hydrogen atom or an alkyl group; R^{r1} represents a hydroxy group, an alkoxy group having 1 to 3 carbon atoms, an amino group, an azide group, -OR¹C≡CH, or -R²R³; R^{r2} represents a hydrogen atom, a hydroxy group, a halogen atom, an alkoxy group, an amino group, an azide group, - OR¹C≡CH, or -R²R³; and A¹ represents an oxygen atom or a sulfur atom).

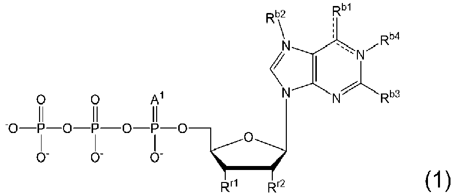

## Description

### [Technical Field]

The present invention relates to a method for capping an RNA (RNA capping method), a method for producing a modified RNA, and a modified RNA.

Priority is claimed on Japanese Patent Application No. 2019-73163, filed April 5, 2019, the content of which is incorporated herein by reference.

### [Background Art]

Since artificial mRNA is unlikely to be integrated into genomic DNA in principle, it is expected to be used as a safe gene carrier in a gene transfer method in clinical research and biological research such as gene therapy and reprogramming of differentiated cells. However, because RNA is an unstable molecule, its effectiveness and persistence time are limited.

Artificial mRNA requires a structure called a 5' cap that controls the initiation of translation and the suppression of degradation. Attempts have been made to increase resistance to degrading enzymes and to improve affinity with translation initiation factors to enhance translational activity by introducing chemical modifications into this 5' cap (for example, Non-Patent Documents 1 and 2).

However, in the methods described in Non-Patent Documents 1 and 2, it is necessary to chemically synthesize a dinucleotide having a modified cap structure for each modification to be introduced. In addition, in the case of synthesis by an in vitro transcription reaction, it is necessary to reduce the amount of GTP because competition with GTP occurs when it is incorporated into the RNA transcription start site. For this reason, there are problems that the amount of obtained RNA is reduced or a certain amount of RNA having no modified cap is produced.

Although a method of producing RNA having an azide group or an amino group at a 5' cap site by the above method and introducing further modifications by a chemical reaction specific to those functional groups has also been reported (Non-Patent Documents 3 and 4), the above problems have not been solved.

In addition, a method of introducing a chemical modification into a guanine base of the 5' cap using methyltransferase and a modified analog of a methyl group donor (SAM) has also been reported (Non-Patent Document 5). However, it has not become a general method for synthesizing RNA with a modified cap, since there are many problems such as the need to prepare methyltransferase and SAM analogs, the limitation of modifications that can be introduced due to the substrate specificity of methyltransferase, and the fact that the translational activity is often reduced.

In addition, an example in which a GTP analog is introduced into a 5' cap of RNA using a capping enzyme derived from chlorella virus PBCV has also been reported (Non-Patent Document 6). However, there were many modified GTPs that could not be introduced into a 5' cap, and the introduction efficiency of the introduced GTP analogs was also not high.

### [Citation List]

### [Non-Patent Documents]

[Non-Patent Document 1] Stepinski J et al., Synthesis and properties of mRNAs containing the novel "anti-reverse" cap analogs 7-methyl (3'-O-methyl) GpppG and 7-methyl (3'-deoxy) GpppG. RNA (2001) 7: 1486-95.
[Non-Patent Document 2] Grudzien-Nogalska E et al., Synthetic mRNAs with superior translation and stability properties. Methods Mol Biol (2013) 969: 55-72.
[Non-Patent Document 3] Mamot A et al., Azido-Functionalized 5' Cap Analogues for the Preparation of Translationally Active mRNAs Suitable for Fluorescent Labeling in Living Cells. Angew Chem Int Ed Engl (2017) 56 (49): 15628-32.
[Non-Patent Document 4] Warminski M et al., Amino-Functionalized 5' Cap Analogs as Tools for Site-Specific Sequence-Independent Labeling of mRNA. Bioconjug Chem (2017) 28 (7): 1978-92.
[Non-Patent Document 5] Muttach F1, et al., Chemo-enzymatic modification of eukaryotic mRNA. Org Biomol Chem (2017) 15 (2): 278-84.
[Non-Patent Document 6] Issur M et al., Enzymatic synthesis of RNAs capped with nucleotide analogues reveals the molecular basis for substrate selectivity of RNA capping enzyme: impacts on RNA metabolism. PLoS One (2013) 8 (9): e75310.

### [Summary of Invention]

### [Technical Problem]

As described above, the types of modification that can be introduced are limited, the introduction efficiency of the modification is also not high, and so on, all of which are problems associated with the conventional method of introducing modifications into a 5' cap site of RNA. Furthermore, there are many cases where the preparation and operation for introducing the modification into the 5' cap site are complicated.

Accordingly, an object of the present invention is to provide an RNA capping method capable of easily introducing various modifications into a 5' cap site, a method for producing a modified RNA using the aforementioned capping method, and a novel modified RNA produced by the aforementioned method for producing a modified RNA.

### [Solution to Problem]

The present invention includes the following aspects.
[1] An RNA capping method including a step of reacting a compound (1) represented by the following general formula (1) (excluding GTP) with an RNA in the presence of a Vaccinia virus capping enzyme. 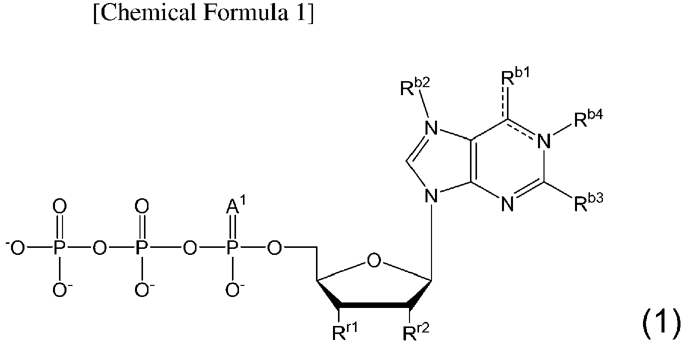 [In the formula, R^{b1} represents an oxo group, an alkoxy group having 1 to 3 carbon atoms, or a halogen atom; R^{b2} is either absent or represents an alkyl group having 1 to 3 carbon atoms; R^{b3} represents an amino group or a hydrogen atom; R^{b4} is either absent or represents a hydrogen atom or an alkyl group having 1 to 3 carbon atoms; R^{r1} represents a hydroxy group, an alkoxy group having 1 to 3 carbon atoms, an amino group, an azide group, -OR¹C≡CH, or -R²R³; R^{r2} represents a hydrogen atom, a hydroxy group, a halogen atom, an alkoxy group having 1 to 3 carbon atoms, an amino group, an azide group, -OR¹C≡CH, or -R²R³; A¹ represents an oxygen atom or a sulfur atom. R¹ represents an alkylene group having 1 to 3 carbon atoms, R² represents -OC(=O)NH-, - OC(=O)-, -NHC(=O)-, -O- or a single bond; and R³ represents an aminoalkyl group having 1 to 3 carbon atoms. A double line composed of a solid line and a dotted line represents a single bond or a double bond.]
[2] The capping method according to [1], wherein the compound (1) is selected from the group consisting of N7-methylguanosine-5'-triphosphate, N1-methylguanosine-5'-triphosphate, O6-methylguanosine-5'-triphosphate, 6-chloroguanosine-5' -triphosphate, inosine-5'-triphosphate, 2'-deoxyguanosine-5'-triphosphate, araguanosine-5'-triphosphate, 2' -fluoro-2' -deoxyguanosine-5' -triphosphate, 2'-O-methylguanosine-5'-triphosphate, 3'-O-methylguanosine-5'-triphosphate, 2'-amino-2'-deoxyguanosine-5'-triphosphate, 3'-amino-2',3'-dideoxyguanosine-5'-triphosphate, 2'-azido-2'-deoxyguanosine-5' -triphosphate, 3'-azido-2',3'-dideoxyguanosine-5'-triphosphate, 3'-(O-propargyl)-guanosine-5'-triphosphate, 2'/3'-O-(2-aminoethyl-carbamoyl)-guanosine-5'-triphosphate and guanosine-5'-O-(1-thiotriphosphate).
[3] A method for producing a modified RNA, including a step (a) of reacting a compound (1) represented by the following general formula (1) (excluding GTP) with an RNA in the presence of a Vaccinia virus capping enzyme. 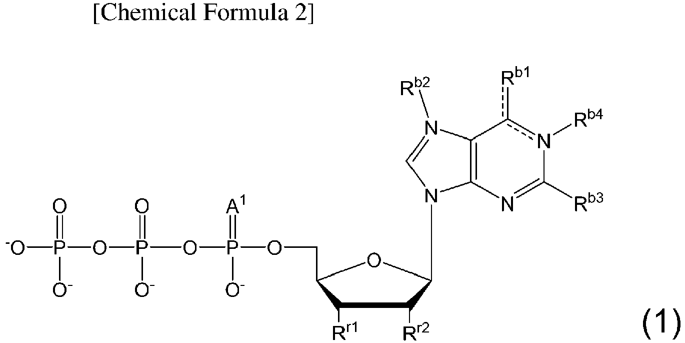 [In the formula, R^{b1} represents an oxo group, an alkoxy group having 1 to 3 carbon atoms, or a halogen atom; R^{b2} is either absent or represents an alkyl group having 1 to 3 carbon atoms; R^{b3} represents an amino group or a hydrogen atom; R^{b4} is either absent or represents a hydrogen atom or an alkyl group having 1 to 3 carbon atoms; R^{r1} represents a hydroxy group, an alkoxy group having 1 to 3 carbon atoms, an amino group, an azide group, -OR¹C≡CH, or -R²R³; R^{r2} represents a hydrogen atom, a hydroxy group, a halogen atom, an alkoxy group having 1 to 3 carbon atoms, an amino group, an azide group, -OR¹C≡CH, or -R²R³; A¹ represents an oxygen atom or a sulfur atom. R¹ represents an alkylene group having 1 to 3 carbon atoms, R² represents -OC(=O)NH-, - OC(=O)-, -NHC(=O)-, -O- or a single bond; and R³ represents an aminoalkyl group having 1 to 3 carbon atoms. A double line composed of a solid line and a dotted line represents a single bond or a double bond.]
[4] The method for producing a modified RNA according to [3], wherein the compound (1) is selected from the group consisting of N7-methylguanosine-5'-triphosphate, N1-methylguanosine-5'-triphosphate, O6-methylguanosine-5'-triphosphate, 6-chloroguanosine-5' -triphosphate, inosine-5' -triphosphate, 2' -deoxyguanosine-5' - triphosphate, araguanosine-5' -triphosphate, 2' -fluoro-2' -deoxyguanosine-5' -triphosphate, 2' -O-methylguanosine-5' -triphosphate, 3' -O-methylguanosine-5' -triphosphate, 2' - amino-2'-deoxyguanosine-5'-triphosphate, 3'-amino-2',3'-dideoxyguanosine-5'-triphosphate, 2'-azido-2'-deoxyguanosine-5'-triphosphate, 3'-azido-2',3'-dideoxyguanosine-5'-triphosphate, 3'-(O-propargyl)-guanosine-5'-triphosphate, 2'/3'-O-(2-aminoethyl-carbamoyl)-guanosine-5'-triphosphate and guanosine-5'-O-(1-thiotriphosphate).
[5] The method for producing a modified RNA according to [3], further including, after the step (a), a step (b1) of reacting an alkyne compound, wherein R^{r1} or R^{r2} in the general formula (1) is an azide group.
[6] The method for producing a modified RNA according to [5], wherein the compound (1) is 2'-azido-2'-deoxyguanosine-5'-triphosphate or 3'-azido-2',3'-dideoxyguanosine-5' -triphosphate.
[7] The method for producing a modified RNA according to [5] or [6], wherein the alkyne compound contains a functional group.
[8] The method for producing a modified RNA according to [3], further including, after the step (a), a step (b2) of reacting an azide compound, wherein R^{r1} or R^{r2} in the general formula (1) is -OR¹C≡CH.
[9] The method for producing a modified RNA according to [8], wherein the compound (1) is 3'-(O-propargyl)-guanosine-5'-triphosphate.
[10] The method for producing a modified RNA according to [8] or [9], wherein the azide compound contains a functional group.
[11] The method for producing a modified RNA according to [3], further including a step (c1) of binding a compound (Ak-1) represented by the following general formula (Ak-1) to a 3' end of the RNA, wherein R^{r1} or R^{r2} in the general formula (1) is an azide group. 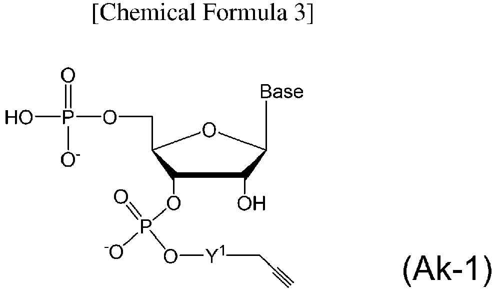 [In the formula, Y¹ represents a divalent linking group; and Base represents a nucleotide base.]
[12] The method for producing a modified RNA according to [11], further including, after the step (a) and the step (c1), a step (d1) of producing a circular RNA by reacting an azide group introduced into a 5' end of the RNA by the step (a) with an alkynyl group introduced into a 3' end of the RNA by the step (c1).
[13] The method for producing a modified RNA according to [11] or [12], wherein the compound (1) is 2'-azido-2'-deoxyguanosine-5'-triphosphate or 3'-azido-2',3'-dideoxyguanosine-5'-triphosphate.
[14] The method for producing a modified RNA according to [3], further including a step (c2) of binding a compound (Az-1) represented by the following general formula (Az-1) to a 3' end of the RNA, wherein R^{r1} or R^{r2} in the general formula (1) is - OR¹C≡CH. 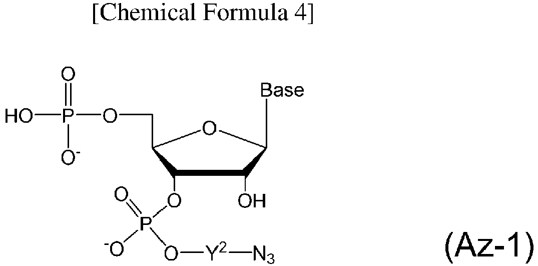 [In the formula, Y² represents a divalent linking group; and Base represents a nucleotide base.]
[15] The method for producing a modified RNA according to [14], further including, after the step (a) and the step (c2), a step (d2) of producing a circular RNA by reacting an alkynyl group introduced into a 5' end of the RNA by the step (a) with an azide group introduced into a 3' end of the RNA by the step (c2).
[16] The method for producing a modified RNA according to [14] or [15], wherein the compound (1) is 3'-(O-propargyl)-guanosine-5'-triphosphate.
[17] A modified RNA including a structure selected from the group consisting of the following formulas (Cp-1) to (Cp-13) at a 5' end. 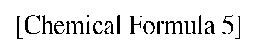 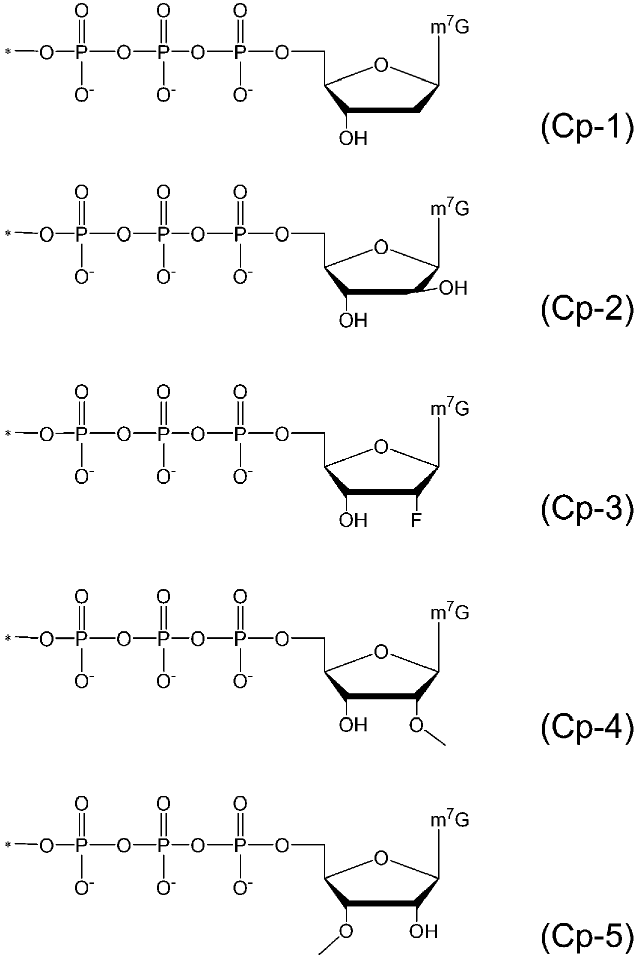 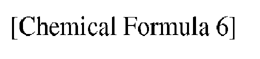 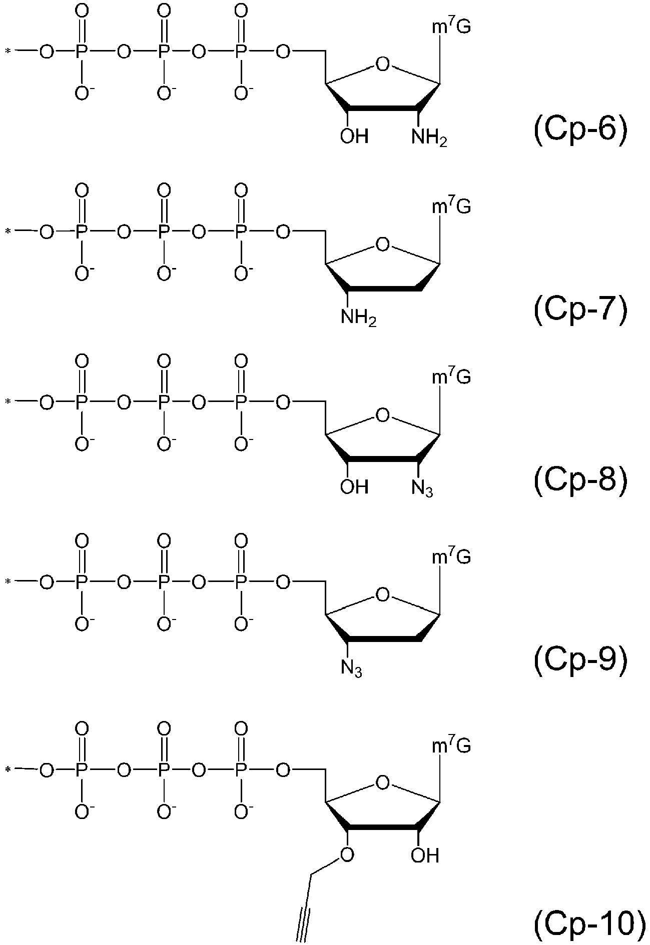 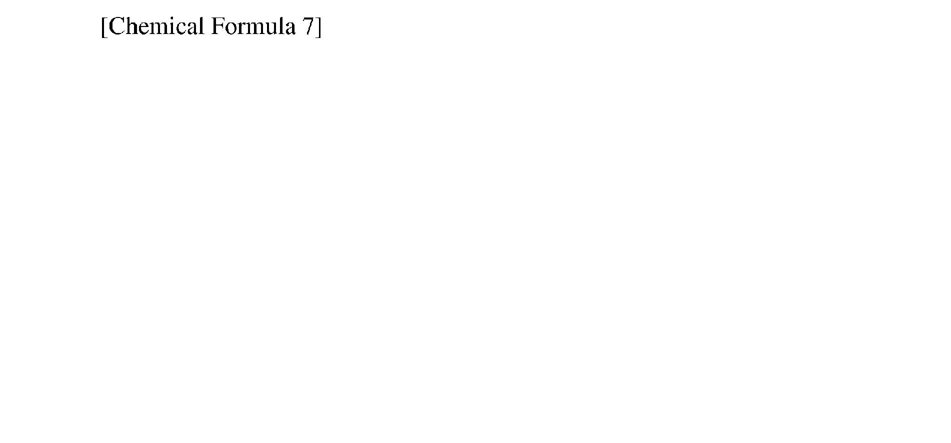 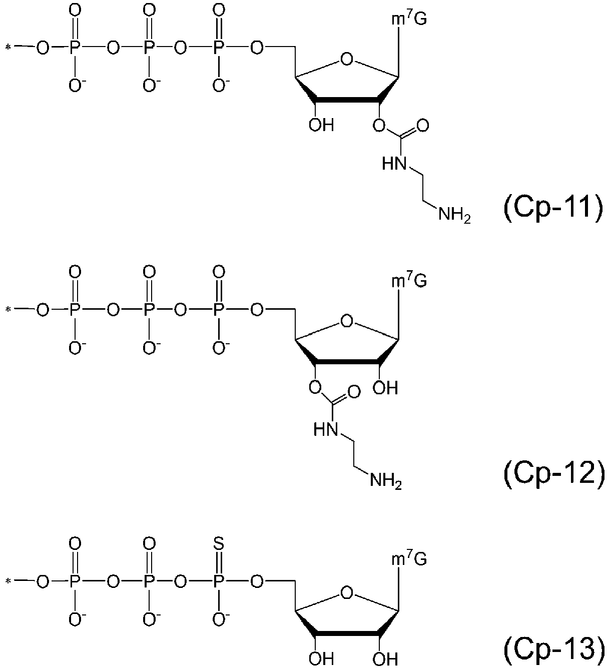 [In each formula, m⁷G represents N7-methylguanine; and ^{∗} represents a bond that binds to a carbon atom at a 5'position of an adjacent nucleotide residue.]
[18] A modified RNA including a structure selected from the group consisting of the following formulas (Ca-1) to (Ca-3) at a 5' end. 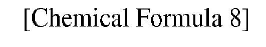 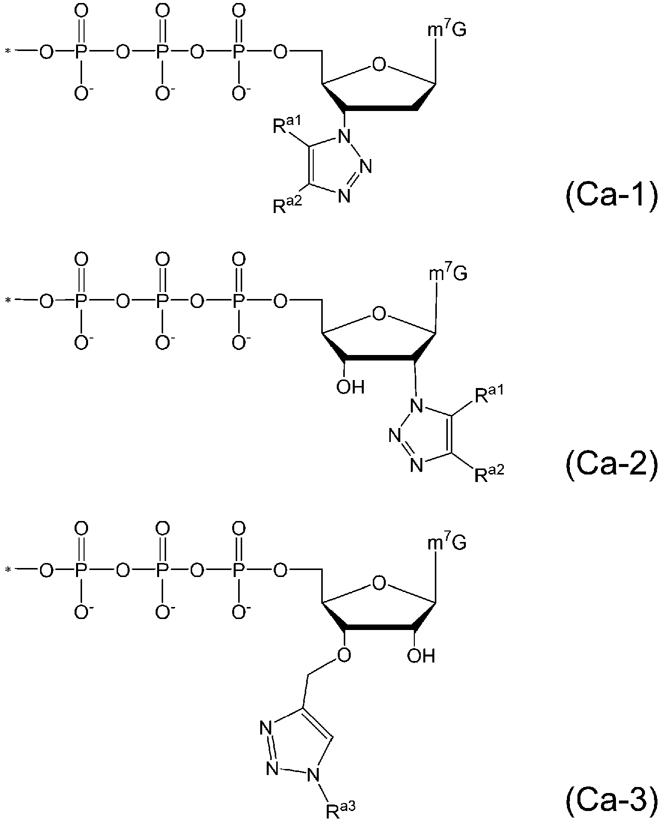 [In each formula, m⁷G represents N7-methylguanine; and ^{∗} represents a bond that binds to a carbon atom at a 5'position of an adjacent nucleotide residue; R^{a1} and R^{a2} each independently represent a hydrogen atom or an organic group, provided that at least one of R^{a1} and R^{a2} is an organic group. When both R^{a1} and R^{a2} are organic groups, they may bind with each other to form a ring structure. R^{a3} represents an organic group.]
[19] A modified circular RNA including a structure selected from the group consisting of the following formulas (Ci-1) to (Ci-3). 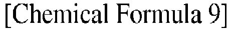 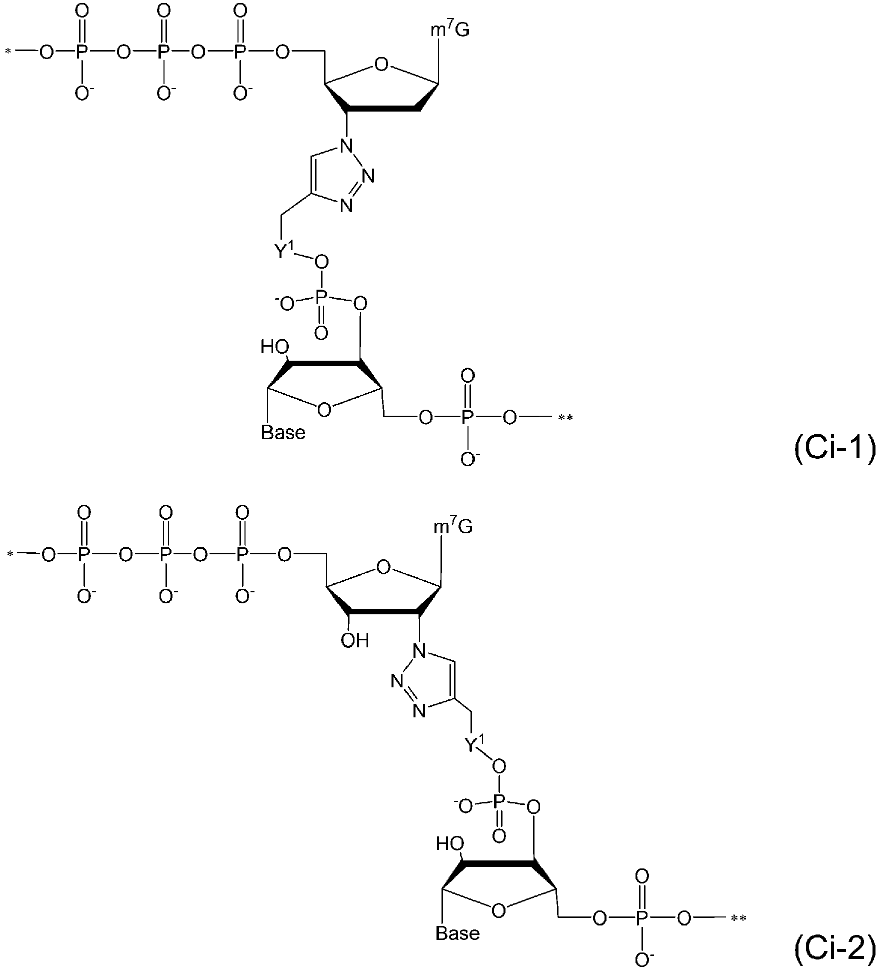 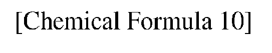 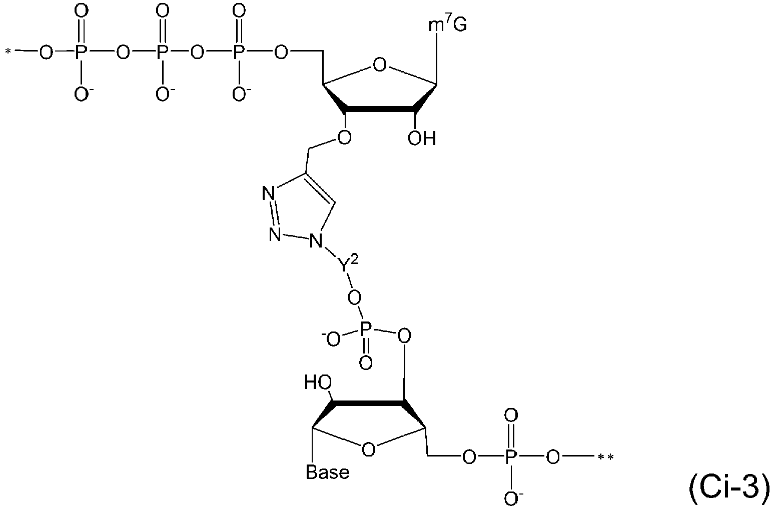 [In the formula, m⁷G represents N7-methylguanine; Y¹ and Y² each independently represent a divalent linking group; Base each independently represents a nucleotide base, ^{∗} represents a bond that binds to a carbon atom at a 5'position of an adjacent nucleotide residue, and ^{∗∗} represents a bond that binds to a carbon atom at a 3'position of an adjacent nucleotide residue.]

### [Advantageous Effects of Invention]

According to the present invention, there are provided an RNA capping method capable of easily introducing various modifications into a 5' cap site, a method for producing a modified RNA using the aforementioned capping method, and a novel modified RNA produced by the aforementioned method for producing a modified RNA.

### [Brief Description of Drawings]

FIG. 1 shows an RNA capping reaction by GTP using a Vaccinia virus capping enzyme (VCE). VCE adds GTP (above) to a 5' end of a transcript and transfers a methyl group of S-adenosylmethionine (SAM) to a 7-position of guanine, thereby producing an RNA with an N7-methylguanosine (m⁷G) cap.
FIG. 2 shows modification sites of GTP analogs used in the examples. They were classified into "Base", "Ribose", "Base and ribose" and "Phosphate" in accordance with the modification sites. In FIG. 2, only structures of the modification sites are shown.
FIG. 3 is a diagram showing a result of evaluating the capping efficiency of each GTP analog in Example 1. The capping efficiency of each GTP analog was calculated from the amount of RNA to which the GTP analog was added and the amount of unreacted RNA.
FIG. 4 is a diagram showing the capping efficiency of each GTP analog when a capping reaction was carried out by doubling the concentration of the GTP analog or doubling the concentrations of the GTP analog and VCE for the GTP analogs having low capping efficiencies in FIG. 3.
FIG. 5A shows a result of evaluating intracellular translational activity of mRNA capped with each GTP analog in Example 2. It is fluorescence microscope images (scale bar: 200 µm) of HeLa cells transfected with mRNA capped with each GTP analog.
FIG. 5B shows a result of evaluating translational activity of mRNA capped with each GTP analog in HeLa cells in Example 2. For cells expressing a red fluorescent protein iRFP670 cotransfected as a transfection control, an average value of the Azami-Green fluorescence level measured with a flow cytometer was corrected by the expression level of iRFP670 determined in a similar manner.
FIG. 6A shows a result of evaluating intracellular translational activity of mRNA capped with each GTP analog in Example 2. It is fluorescence microscope images (scale bar: 200 µm) of 293FT cells transfected with mRNA capped with each GTP analog.
FIG. 6B shows a result of evaluating translational activity of mRNA capped with each GTP analog in 293FT cells in Example 2. For cells expressing the red fluorescent protein iRFP670 cotransfected as a transfection control, an average value of the Azami-Green fluorescence level measured with a flow cytometer was corrected by the expression level of iRFP670 determined in a similar manner.
FIG. 7 shows chemical structural formulas of DBCO-biotin and DBCO-AF647 used in Example 3.
FIG. 8 is a diagram showing a reaction scheme in which a cap having an azide group is modified with DBCO-dye/biotin using a SPAAC reaction.
FIG. 9 is a diagram showing a result of introducing DBCO-dye/biotin into RNA having an azide group in a cap by a SPAAC reaction in Example 3.
FIG. 10 is a denatured PAGE gel photograph showing that DBCO-AF647 has been introduced into a cap having an azide group of mRNA produced in Example 3.
FIG. 11 is a confocal fluorescence microscope image of HeLa cells transfected with AF647-labeled mRNA in Example 3. The left end column shows bright field images, and the three columns to the right thereof show observations of fluorescence of the nucleus (stained with Hoechst), Azami-Green (green fluorescent protein), and AF647 (red fluorescent dye), respectively. In the figure, "Mock" indicates that a transfection treatment was carried out without RNA, and "Untreated" indicates that a transfection treatment was not carried out.
FIG. 12 is a diagram illustrating a scheme for circular RNA production in Example 4.
FIG. 13 is a diagram showing a result of denatured PAGE in Example 4.

### [Description of Embodiments]

In the present specification and claims, depending on a structure represented by a chemical formula, an asymmetric carbon may be present, and an enantiomer or a diastereomer may be present. In that case, one chemical formula is used to represent those isomers. These isomers may be used alone or as a mixture.

### [RNA capping method]

In one embodiment, the present invention provides a method for capping RNA, which includes a step of reacting an RNA with a compound (1) represented by the following general formula (1) (excluding GTP) in the presence of a vaccinia virus capping enzyme. 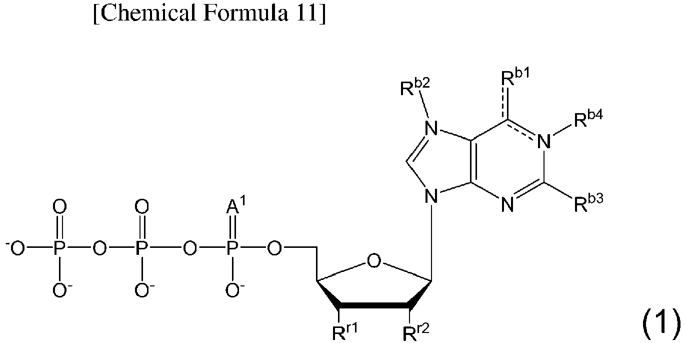 [In the formula, R^{b1} represents an oxo group, an alkoxy group having 1 to 3 carbon atoms, or a halogen atom; R^{b2} is either absent or represents an alkyl group having 1 to 3 carbon atoms; R^{b3} represents an amino group or a hydrogen atom; R^{b4} is either absent or represents a hydrogen atom or an alkyl group having 1 to 3 carbon atoms; R^{r1} represents a hydroxy group, an alkoxy group having 1 to 3 carbon atoms, an amino group, an azide group, -OR¹C≡CH, or -R²R³; R^{r2} represents a hydrogen atom, a hydroxy group, a halogen atom, an alkoxy group having 1 to 3 carbon atoms, an amino group, an azide group, -OR¹C≡CH, or -R²R³; A¹ represents an oxygen atom or a sulfur atom. R¹ represents an alkylene group having 1 to 3 carbon atoms, R² represents -OC(=O)NH-, - OC(=O)-, -NHC(=O)-, -O- or a single bond; and R³ represents an aminoalkyl group having 1 to 3 carbon atoms. A double line composed of a solid line and a dotted line represents a single bond or a double bond.]

In a capping method according to the present embodiment, a capping reaction is carried out using a vaccinia virus capping enzyme. As shown in the examples described later, the vaccinia virus capping enzyme is applicable to a wide range of GTP analogs and various modifications can be introduced into an RNA cap site. The structure of the cap site affects RNA stability, translational activity, and the like. For this reason, by capping RNA by the capping method according to the present embodiment, various RNAs having different degrees of stability and translational activity can be obtained.

### <Vaccinia virus capping enzyme: VCE>

A "vaccinia virus capping enzyme" is a capping enzyme possessed by a vaccinia virus (Martin SA, et al., J Biol Chem (1976) 251 (23): 7313-7321.; Fuchs AL, et al., RNA (2016) 22 (9): 1454-1466.). A capping enzyme is an enzyme that can add a cap to the 5' end of RNA. The cap has a structure in which guanosine or a guanosine derivative is bound to the 5' end of RNA via a triphosphate bond, and is involved in intracellular stabilization of RNA, initiation of translation, and the like. The vaccinia virus capping enzyme is composed of two subunits, a large subunit (Gene ID: 3707562) and a small subunit (Gene ID: 3707515).

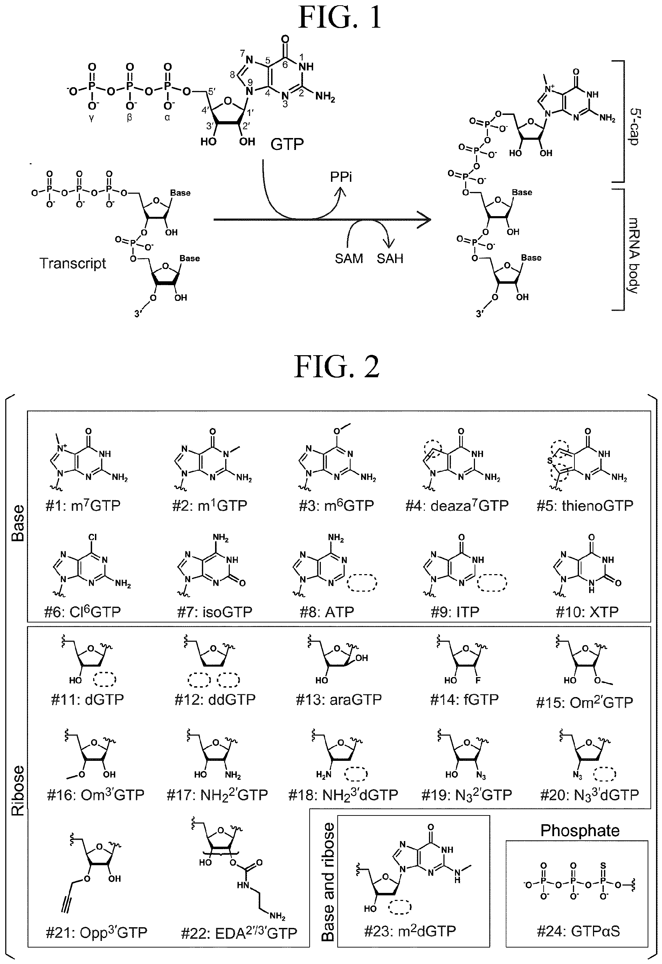

FIG. 1 is a diagram schematically showing an RNA capping reaction by a capping enzyme. When GTP is reacted with RNA in the presence of a capping enzyme, GMP is transferred and bound to the 5' end of RNA from GTP, and pyrophosphate (PPi) is separated. Furthermore, a methyl group is transferred from S-adenosylmethionine (SAM) to a nitrogen atom at a 7-position of a guanine base, and a cap containing a base of N7-methylguanine (m⁷G) is formed at the 5' end of RNA. In FIG. 1, by using a GTP analog (modified GTP) instead of GTP, caps having various structures can be introduced into the 5' end of RNA.

The vaccinia virus capping enzyme is known, and the sequence information can be obtained from a known database such as GenBank. Examples of the base sequences of the large subunit and the small subunit of the vaccinia virus capping enzyme gene include the sequences set forth in SEQ ID NO: 2 and SEQ ID NO: 4, respectively. Further, examples of the amino acid sequences of the large subunit and the small subunit of the vaccinia virus capping enzyme include the sequences set forth in SEQ ID NO: 3 (NCBI Reference Sequence: YP_232988.1) and SEQ ID NO: 5 (NCBI Reference Sequence: YP_232999.1), respectively. For example, a vaccinia virus capping enzyme gene can be obtained by designing an appropriate primer based on the sequence information and performing PCR or the like using vaccinia virus DNA as a template. Furthermore, the vaccinia virus capping enzyme can be obtained by introducing the enzyme gene into an appropriate cell, expressing it, and purifying it from the cell using a known enzyme purification means. A cell-free protein synthesis system may be used for the synthesis of the capping enzyme from the enzyme gene.

In addition, a commercially available product may be used for the vaccinia virus capping enzyme. Examples of commercially available products include the ScriptCap m⁷G Capping System (CellScript).

Hereinafter, the vaccinia virus capping enzyme may be referred to as "VCE".

### <Compound (1)>

A compound (1) is a compound represented by the above general formula (1). In a capping method according to the present embodiment, the compound (1) is used in place of GTP and is introduced as a cap at the 5' end of RNA. Although the compounds represented by the above general formula (1) include GTP, GTP is excluded from the compound (1). That is, in the general formula (1), there is no combination in which R^{b1} is an oxo group, R^{b2} is absent, R^{b3} is an amino group, R^{b4} is a hydrogen atom, R^{r1} is a hydroxy group, R^{r2} is a hydroxy group, and A' is an oxygen atom.

In the above general formula (1), R^{b1} represents an oxo group, an alkoxy group having 1 to 3 carbon atoms, or a halogen atom. Examples of the halogen atom include a fluorine atom, a chlorine atom, a bromine atom and an iodine atom, and among them, a chlorine atom is preferable. Examples of the alkoxy group having 1 to 3 carbon atoms include a methoxy group, an ethoxy group, and a propoxy group, and a methoxy group or an ethoxy group is preferable, and a methoxy group is more preferable.

In the above general formula (1), R^{b2} is either absent or represents an alkyl group having 1 to 3 carbon atoms. Examples of the alkyl group having 1 to 3 carbon atoms include a methyl group, an ethyl group, and a propyl group, and a methyl group or an ethyl group is preferable, and a methyl group is more preferable. When R^{b2} is an alkyl group having 1 to 3 carbon atoms, a nitrogen atom to which R^{b2} binds in the general formula (1) is positively charged.

In the above general formula (1), R^{b3} represents an amino group or a hydrogen atom.

In the above general formula (1), R^{b4} is either absent or represents a hydrogen atom or an alkyl group having 1 to 3 carbon atoms. Examples of the alkyl group having 1 to 3 carbon atoms include a methyl group, an ethyl group, and a propyl group, and a methyl group or an ethyl group is preferable, and a methyl group is more preferable.

In the above general formula (1), R^{r1} represents a hydroxy group, an alkoxy group having 1 to 3 carbon atoms, an amino group, an azide group, -OR¹C≡CH, or -R²R³.

Examples of the alkoxy group having 1 to 3 carbon atoms include a methoxy group, an ethoxy group, and a propoxy group, and a methoxy group or an ethoxy group is preferable, and a methoxy group is more preferable.

R¹ in the above -OR¹C≡CH represents an alkylene group having 1 to 3 carbon atoms. Examples of the alkylene group having 1 to 3 carbon atoms include a methylene group, an ethylene group, and a propylene group, and a methylene group or an ethylene group is preferable, and a methylene group is more preferable.

R² in the above -R²R³ represents -OC(=O)NH-, -OC(=O)-, -NHC(=O)-, -O- or a single bond. R² is preferably -OC(=O)NH- or a single bond, and more preferably - OC(=O)NH-. R³ in the above -R²R³ represents an aminoalkyl group having 1 to 3 carbon atoms. Examples of the aminoalkyl group having 1 to 3 carbon atoms include an aminomethyl group, an aminoethyl group, and an aminopropyl group, and an aminomethyl group or an aminoethyl group is preferable, and an aminoethyl group is more preferable. -R²R³ is particularly preferably an aminoethylcarbamoyloxy group.

In the above general formula (1), R^{r2} represents a hydrogen atom, a hydroxy group, a halogen atom, an alkoxy group having 1 to 3 carbon atoms, an amino group, an azide group, -OR¹C≡CH, or -R²R³. Examples of the halogen atom include a fluorine atom, a chlorine atom, a bromine atom and an iodine atom, and among them, a fluorine atom is preferable. R¹ in -OR¹C≡CH is the same as described above. R² and R³ in R²R³ are the same as described above, respectively.

In the above general formula (1), A¹ represents an oxygen atom or a sulfur atom.

In the above general formula (1), a double line composed of a solid line and a dotted line represents a single bond or a double bond.

When a base portion in the general formula (1) is represented as Base and a sugar portion is represented as Sugar, the compound (1) can be represented by the following general formula (1').

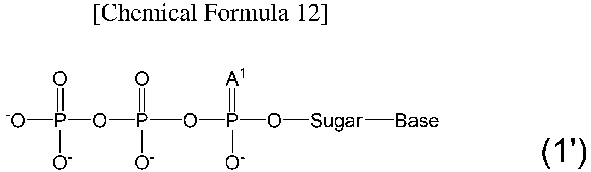

Specific examples of Base in the above general formula (1') include bases represented by any of the following formulas (B-1) to (B-5). In the formulas, ^{∗} represents a bond that binds to Sugar in the formula (1').

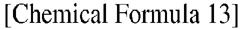

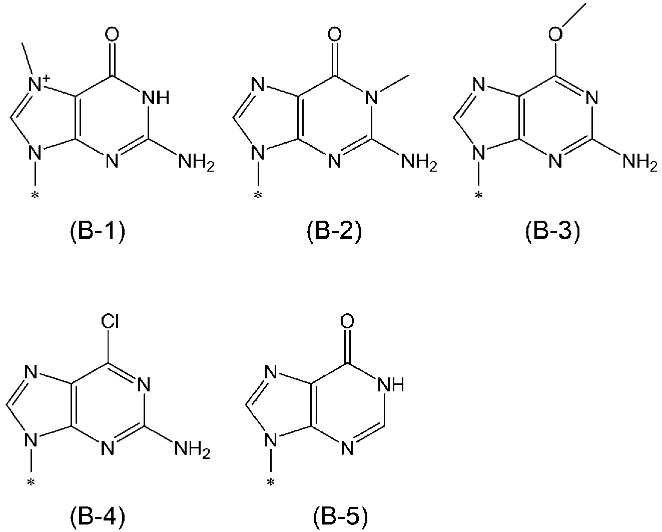

Specific examples of Sugar in the above general formula (1') include bases represented by any of the following formulas (R-1) to (R-12). In the formulas, ^{∗} represents a bond that binds to Base in the formula (1'). ^{∗∗} represents a bond that binds to an oxygen atom in the formula (1').

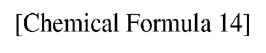

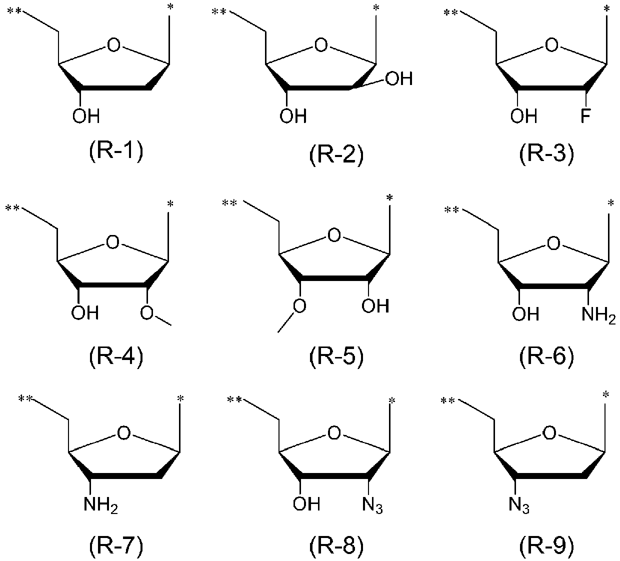

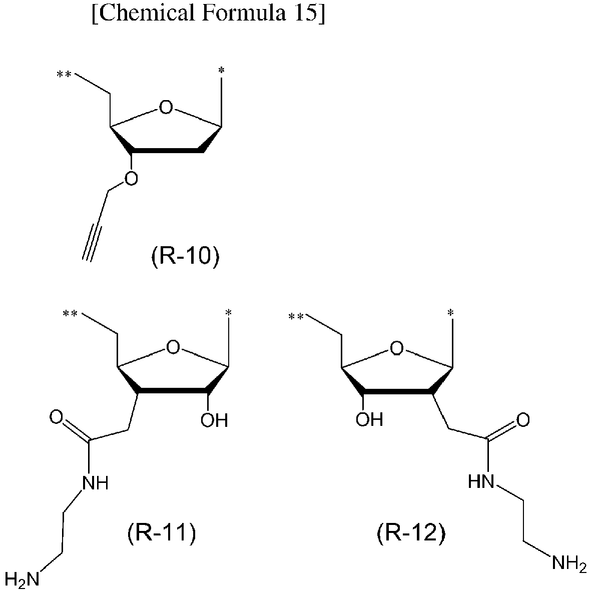

Specific examples of the preferred compound (1) include N7-methylguanosine-5'-triphosphate (hereinafter, also referred to as "m⁷GTP"), N1-methylguanosine-5'-triphosphate (hereinafter, also referred to as "m¹GTP"), O6-methylguanosine-5'-triphosphate (hereinafter, also referred to as "m⁶GTP"), 6-chloroguanosine-5'-triphosphate (hereinafter, also referred to as "Cl⁶GTP"), inosine-5'-triphosphate (hereinafter, also referred to as "ITP"), 2'-deoxyguanosine-5'-triphosphate (hereinafter, also referred to as "dGTP"), araguanosine-5'-triphosphate (hereinafter, also referred to as "araGTP"), 2'-fluoro-2'-deoxyguanosine-5'-triphosphate (hereinafter, also referred to as "fGTP"), 2'-O-methylguanosine-5'-triphosphate (hereinafter, also referred to as "Om^{2'}GTP"), 3'-O-methylguanosine-5'-triphosphate (hereinafter, also referred to as "Om^{3'}GTP"), 2'-amino-2'-deoxyguanosine-5'-triphosphate (hereinafter, also referred to as "NH₂²'GTP"), 3'-amino-2',3'-dideoxyguanosine-5'-triphosphate (hereinafter, also referred to as "NH₂^{3'}dGTP"), 2'-azido-2'-deoxyguanosine-5'-triphosphate (hereinafter, also referred to as "N₃^{2'}GTP"), 3'-azido-2',3'-dideoxyguanosine-5'-triphosphate (hereinafter, also referred to as "N₃^{3'} dGTP'), 3'-(O-propargyl)-guanosine-5'-triphosphate (hereinafter, also referred to as "Opp^{3'}GTP"), 2'/3'-O-(2-aminoethyl-carbamoyl)-guanosine-5'-triphosphate (hereinafter, also referred to as "EDA^{2'}/^{3'}GTP"), and guanosine-5'-O-(1-thiotriphosphate) (hereinafter, also referred to as "GTPαS"). EDA^{2'}/^{3'}GTP is a mixture of compounds in which the 2' or 3' position of sugar is substituted with an O-(2-aminoethyl-carbamoyl) group.

Among these, from the viewpoint of capping efficiency, m⁷GTP, m¹GTP, m⁶GTP, Cl⁶GTP, dGTP, araGTP, fGTP, Om^{2'}GTP, Om^{3'}GTP, NH₂^{2'}GTP, NH₂^{3'}dGTP, N₃^{2'}GTP, N₃^{3'}dGTP, Opp^{3'}GTP, EDA^{2'}/^{3'}GTP, and GTPαS are preferable.

For the compound (1), a commercially available product can be used.

### <RNA>

RNA is not particularly limited and may have any sequence and strand length, but one with no cap at the 5' end is used.

RNA can be synthesized by a known method. For example, RNA can be obtained by transcribing mRNA from template DNA by an in vitro method using T7 RNA polymerase. In this case, it is preferable to introduce a promoter sequence (T7 promoter sequence) that can be recognized by the T7 RNA polymerase into the template DNA. As an in vitro RNA transcription system, various commercially available products (for example, MEGAscript T7 Transcription Kit, Thermo Fisher Scientific, and the like) are available. For this reason, RNA may be synthesized from the template DNA using these commercially available products. The synthesized RNA can be purified using a commercially available RNA purification kit (for example, RNeasy MinElute Cleanup Kit, Qiagen) or the like after removing DNA by a DNase treatment or the like.

Further, in the case of short strand RNA (for example, 100 bases long or less), it may be synthesized by a known RNA synthesis method such as a phosphoramidite method.

### <Reaction step>

A capping method according to the present embodiment includes a step of reacting the compound (1) with RNA in the presence of VCE. The above reaction can be carried out under conditions generally used for the capping reaction. For example, VCE, the compound (1) and RNA are added to a suitable buffer solution and reacted at 30 to 38°C, preferably 35 to 37°C. The reaction time is not particularly limited, but for example, 30 minutes to 50 hours can be exemplified, and 1 to 30 hours are preferable. Since the preferable reaction time varies depending on the type of the compound (1) to be used, the reaction time may be set in accordance with the type of the compound (1) to be used. For example, in those cases where m⁷GTP, m¹GTP, m⁶GTP, dGTP, araGTP, Om^{3'}GTP, NH₂^{2'}GTP, NH₂^{3'}dGTP, N₃^{3'}dGTP, Opp^{3'}GTP, or EDA^{2'}/^{3'}GTP is used as the compound (1), the reaction time can be set to about 30 to 120 minutes, preferably about 50 to 70 minutes. On the other hand, when Cl⁶GTP, ITP, fGTP, Om^{2'}GTP, N₃^{2'}GTP, or GTPαS is used as the compound (1), the reaction time can be set to about 10 to 30 hours, preferably about 20 to 30 hours.

In addition to VCE, the compound (1) and RNA, SAM, an RNase inhibitor, and the like can be added to the reaction solution of the capping reaction.

Further, the concentrations of VCE, compound (1) and RNA in the reaction solution can be appropriately set in accordance with the type of the compound (1).

After the capping reaction, uncapped RNA may be removed. The method for removing the uncapped RNA is not particularly limited, but for example, a method in which the 5' end of the uncapped RNA is monophosphorylated by dephosphorylation with an alkaline phosphatase or the like and phosphorylation with a polynucleotide kinase, followed by degradation of monophosphorylated RNA with an exonuclease that specifically degrades 5'-monophosphorylated RNA can be mentioned.

After appropriately removing the uncapped RNA as described above, RNA may be purified using a commercially available RNA purification kit or the like.

The capping method according to the present embodiment can be used in the method for producing a modified RNA described later.

### [Method for producing modified RNA]

In one embodiment, the present invention provides a method for producing a modified RNA, which includes a step (a) of reacting a compound (1) represented by the above general formula (1) (excluding GTP) with RNA in the presence of a capping enzyme of vaccinia virus.

### <Step (a)>

Step (a) can be performed in the same manner as the reaction step in the RNA capping method of the above embodiment. The VCE, compound (1), and RNA are the same as those described above, and the preferred examples thereof are also the same.

A modified RNA obtained by the production method of the present embodiment is RNA in which the compound (1) is introduced as a cap at the 5' end of the RNA. In the present specification, the term "modified RNA" means RNA having a cap with a structure other than that of m⁷G at the 5' end.

Those obtained by removing pyrophosphate from the compound (1) by the step (a) bind to triphosphate at the 5' end of RNA to form a cap. As a result, it is possible to obtain a modified RNA in which a nucleoside structure of the compound (1) is bound to the 5' end via a 5'-5' triphosphate bond.

### <Arbitrary step>

The production method of the present embodiment may include other steps in addition to the above step (a). Examples of other steps include a step of further modifying the modified RNA obtained in the step (a). Further modifications are not particularly limited, and examples thereof include modifications using click chemistry. More specifically, using click chemistry, a step of introducing a functional group into the modified RNA obtained in the step (a), a step of circularizing the modified RNA obtained in the step (a), or the like can be mentioned.

### «Introduction of functional group»

The production method of the present embodiment may further include a step of introducing an alkyne compound or an azide compound after the above step (a). Specific examples of such a step include the following steps (b1) and (b2). Further, by allowing an active molecule to bind to the above alkyne compound or azide compound, a functional group can be introduced into the modified RNA.

### (Step (b1))

For example, in a case where the compound (1) is a compound in which R^{r1} or R^{r2} in the above general formula (1) is an azide group (-N=N+=N-) (hereinafter, also referred to as compound (1-az)), the production method according to the present embodiment can further include a step (b1) of reacting an alkyne compound after the above step (a).

Specific examples of the compound (1-az) include N₃^{2'}GTP and N₃^{3'}dGTP.

In the present specification, the term "alkyne compound" means a compound having a carbon-carbon triple bond (-C=C-) in a molecule. The structure of the alkyne compound is not particularly limited, and may be one obtained by introducing a triple bond into a desired molecule. For example, the alkyne compound can contain a functional group. The term "functional group" means an atomic group having one or more functions, and examples of the functions include, but are not limited to, fluorescence activity, luminescence activity, binding activity, enzyme activity, enzyme inhibitory activity, physiological activity, adhesion activity, and various drug activities. In a compound produced by allowing a functional molecule to bind to an alkyne compound, the functional group may be a group composed of an atomic group originated from the functional molecule (a group derived from the functional molecule). Further, the functional group may be a group derived from a biopolymer such as a protein, sugar or nucleic acid. Specific examples of the functional molecule include, but are not limited to, fluorescent molecules, luminescent molecules, biotin, avidin, streptavidin, enzymes, sugars, polyethylene glycols, steroids, carboxylic acids, amines and succinimides.

When the compound (1) is a compound (1-az), an azide group is introduced into a cap site in the modified RNA obtained by the step (a). In the step (b1), the alkyne compound can be reacted with the above azide group by a Cu-catalyzed azide alkyne cycloaddition (CuAAC) reaction or a strain-promoted azide-alkyne cycloaddition (SPAAC) reaction. For example, when the alkyne compound has a propargyl group, the CuAAC reaction can be used, and the reaction can be carried out in the presence of a copper catalyst. Further, when the alkyne compound is a compound containing a cyclic alkyne structure, the SPAAC reaction can be used, and the reaction can be carried out in the absence of a copper catalyst. The reaction conditions in these reactions are not particularly limited, and the reaction conditions generally used for the CuAAC reaction or SPAAC reaction can be used. For example, these reactions can be carried out at room temperature (about 20 to 38°C), with a reaction time of 30 to 120 minutes, and the like.

### (Step (b2))

For example, when the compound (1) is a compound in which R^{r1} or R^{r2} in the above general formula (1) is -OR¹C≡CH (hereinafter, also referred to as compound (1-pr)), the production method according to the present embodiment can further include a step (b2) of reacting an azide compound after the above step (a).

In the compound (1-pr), the above -OR¹C≡CH is preferably a propargyloxy group. Specific examples of the compound (1-pr) include Opp^{3'} GTP.

In the present specification, the term "azide compound" means a compound having an azide group (-N=N⁺=N⁻) in a molecule. The structure of the azide compound is not particularly limited, and may be one obtained by introducing an azide group into a desired molecule. For example, the azide compound can contain a functional group. Examples of the functional group include the same as those listed above.

When the compound (1) is a compound (1-pr), a propargyl group (-CH₂-C=CH) is introduced into the cap site of the modified RNA obtained by the step (a). In the step (b2), the azide compound can be reacted with the above propargyl group by the CuAAC reaction. Examples of the reaction conditions include the same as those described above.

### <<Formation of circular RNA>>

The production method of the present embodiment may further include, in addition to the above step (a), a step of introducing an alkyne compound or an azide compound into the 3' end of RNA. Specific examples of such a step include the following steps (c1) and (c2). Furthermore, a step of producing a circular RNA may be included after that. Specific examples of such a step include the following steps (d1) and (d2).

### (Step (c1))

When the compound (1) is a compound (1-az), the production method according to the present embodiment can further include a step (c1) of binding a compound represented by the following general formula (Ak-1) (hereinafter, referred to as "compound (Ak-1)") to the 3' end of RNA, in addition to the above step (a). 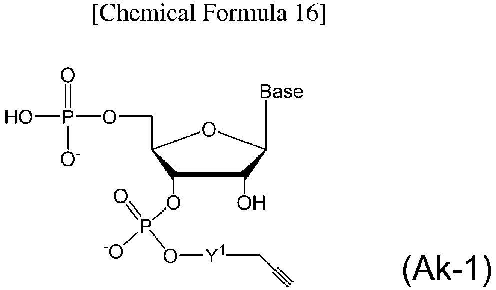 [In the formula, Y¹ represents a divalent linking group; and Base represents a nucleotide base.]

In the above general formula (Ak-1), Y¹ represents a divalent linking group. Examples of the divalent linking group include a hydrocarbon group which may have a substituent. The hydrocarbon group may be an aliphatic hydrocarbon group or an aromatic hydrocarbon group, but an aliphatic hydrocarbon group is preferable. The aliphatic hydrocarbon group preferably has 3 to 50 carbon atoms, more preferably 3 to 30 carbon atoms, and still more preferably 3 to 25 carbon atoms.

The aliphatic hydrocarbon group may be linear, branched, or cyclic, but a linear or branched aliphatic hydrocarbon group is preferable, and a linear aliphatic hydrocarbon group is more preferable. The aliphatic hydrocarbon group may be a saturated aliphatic hydrocarbon group or an unsaturated aliphatic hydrocarbon group, but is preferably a saturated aliphatic hydrocarbon group. Y¹ is preferably a linear saturated aliphatic hydrocarbon group.

The aliphatic hydrocarbon group may have a substituent. The substituent may be one obtained by substituting a hydrogen atom (-H) with a monovalent group, or one obtained by substituting a methylene group with a divalent group. Examples of the monovalent group for substituting the hydrogen atom include, but are not limited to, a hydroxy group, a halogen atom, an amino group and an alkoxy group. Examples of the divalent group for substituting the methylene group include, but are not limited to, -O-, - NHC(=O)-, -NH-, -C(=O)NH-, -C(=O)O-, -OC(=O)-, and -C(-O)-. It is preferable that the hydrogen atom of the aliphatic hydrocarbon group is not substituted, and it is preferable that a portion of the methylene group is substituted with the divalent group. In particular, Y¹ is preferably a linear saturated aliphatic hydrocarbon group in which a portion of the methylene group is substituted with -O- or -NHC(=O)-. Specific examples of Y¹ include -(CH₂)ₘ-NHC(=O)-(CₐH₂ₐO)ₙ-. The above m and n are each independently an integer of 1 or more, and an integer of 1 to 15 can be mentioned. m is preferably an integer of 1 to 10, more preferably an integer of 2 to 8, still more preferably an integer of 3 to 7, particularly preferably 5 or 6, and most preferably 6. The above n is preferably an integer of 1 to 10, more preferably an integer of 2 to 8, still more preferably an integer of 3 to 7, particularly preferably 5 or 6, and most preferably 5. a is an integer of 1 to 3, preferably 1 or 2, and more preferably 2.

In the above general formula (Ak-1), Base represents a nucleotide base. The nucleotide base is selected from the group consisting of guanine, cytosine, adenine, and uracil, as well as their derivatives. The above derivative is not particularly limited as long as the binding to the 3' end of RNA is not inhibited, and a known derivative of guanine, cytosine, adenine or uracil can be used. The nucleotide base is preferably selected from the group consisting of guanine, cytosine, adenine and uracil, and is more preferably cytosine.

Specific examples of the compound (Ak-1) include pCp-alkyne.

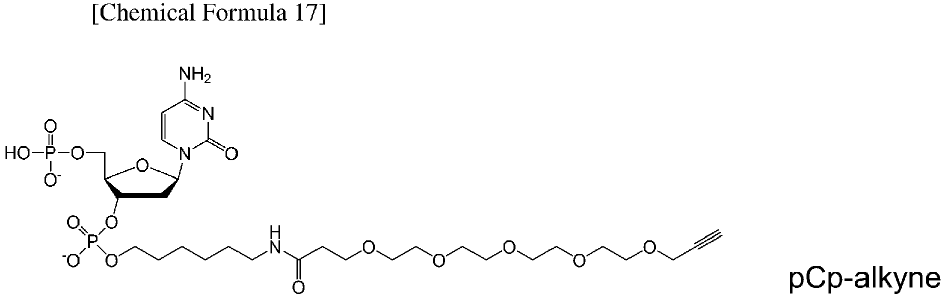

Specific examples of the compound (1-az) include N₃^{2'}GTP and N₃^{3'}dGTP.

Binding of the compound (Ak-1) to the 3' end of RNA can be performed in the presence of T4 RNA ligase. T4 RNA ligase is an enzyme that binds a phosphate group at the 5' end of a nucleic acid to a hydroxy group at the 3' end, and various commercially available products (for example, Ambion (registered trademark) T4 RNA Ligase, and the like) can be used. The binding reaction (ligation reaction) of the compound (Ak-1) to the 3' end of RNA by T4 RNA ligase is preferably carried out in the presence of ATP. When using commercially available T4 RNA ligase, the ligation reaction with T4 RNA ligase may be carried out in accordance with the conditions recommended for the product. For example, RNA, compound (Ak-1), T4 RNA ligase and ATP are mixed in a suitable buffer solution to carry out a ligation reaction. Examples of the reaction temperature include 3 to 20°C, and examples of the reaction time include 16 to 96 hours.

After the ligation reaction, RNA may be purified using a commercially available RNA purification kit or the like.

The step (c1) may be performed after the above step (a) as shown in the following Scheme (c1-1). Alternatively, as shown in the following Scheme (c1-2), it may be performed before the step (a). It should be noted that in the following Scheme, a case where the compound (1) is N₃^{3'}dGTP is described as an example. In the following Scheme, a thick line (from 5' to 3') indicates an RNA strand.

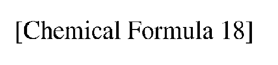

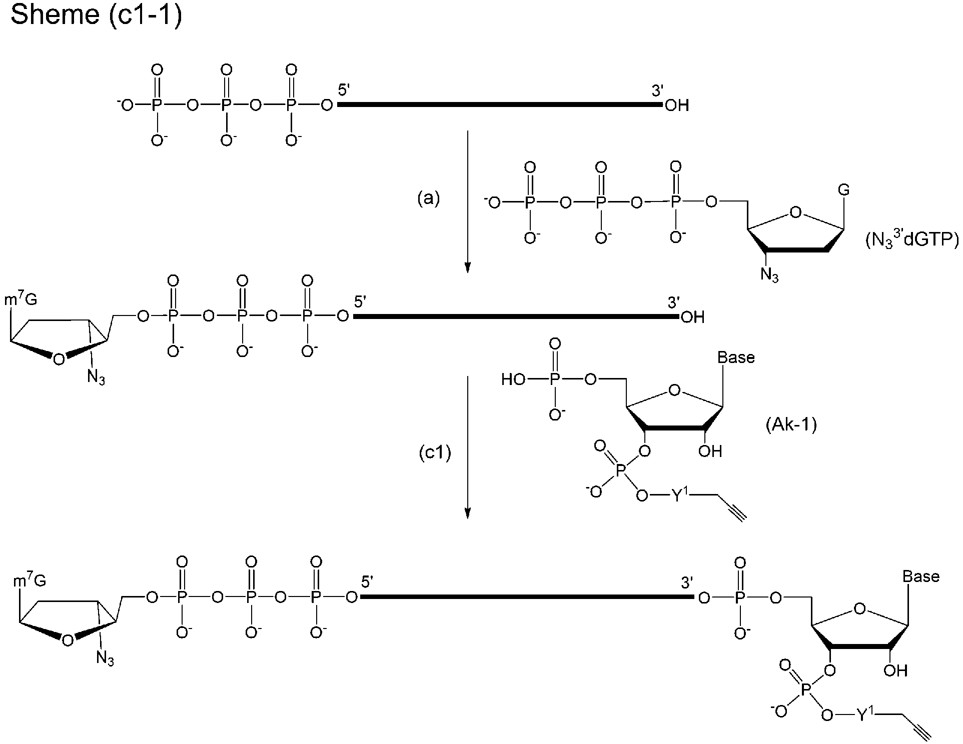

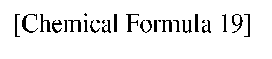

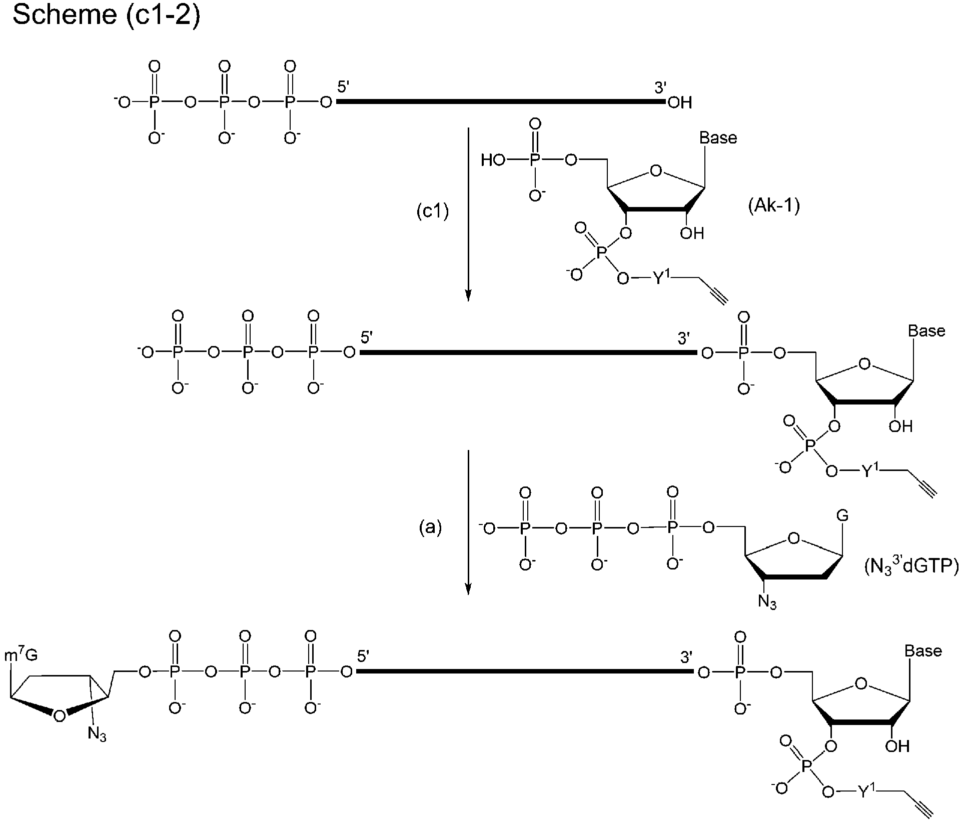

By carrying out the step (c1), an alkynyl group can be introduced into the 3' end of RNA.

### (Step (d1))

The production method according to the present embodiment may further include, after the above steps (a) and (c1), a step (d1) of producing a modified circular RNA by reacting an azide group introduced into the 5' end of RNA by the above step (a) with an alkynyl group introduced into the 3' end of RNA by the above step (c1).

The reaction between the azide group and the alkynyl group can be carried out by the above-mentioned CuAAC reaction. The reaction conditions in the CuAAC reaction are not particularly limited, and the reaction conditions generally used for the CuAAC reaction can be used. For example, the reaction can be carried out at room temperature (about 20 to 38°C), with a reaction time of 30 to 120 minutes, and the like.

A circular RNA can be obtained by binding the azide group at the 5' end of RNA and the alkynyl group at the 3' end by the CuAAC reaction. Since the circular RNA does not have a free end, it is not degraded by exonucleases and is expected to improve its stability in vivo.

### (Step (c2))

When the compound (1) is a compound (1-pr), the production method according to the present embodiment can further include a step (c2) of binding a compound represented by the following general formula (Az-1) (hereinafter, referred to as "compound (Az-1)") to the 3' end of RNA, in addition to the above step (a). 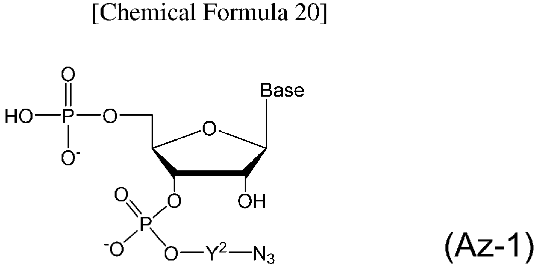 [In the formula, Y² represents a divalent linking group; and Base represents a nucleotide base.]

In the above general formula (Az-1), Y² represents a divalent linking group. Examples of the divalent linking group include the same divalent linking groups as those represented by Y¹ in the above general formula (Ak-1), and the preferred examples thereof are also the same. In particular, Y² is preferably a linear saturated aliphatic hydrocarbon group in which a portion of the methylene group is substituted with -O- or-NHC(=O)-. Specific examples of Y² include -(CH₂)ₘ-NHC(=O)-(CₐH₂ₐO)ₙ-(CH₂)₂-. The above m and n are the same as those described for Y¹ in the above general formula (Ak-1). In particular, m is preferably 5 or 6, and more preferably 6. n is preferably an integer of 3 to 7, more preferably an integer of 3 to 6, and still more preferably 4. The above a is the same as that described for Y¹ in the above general formula (Ak-1). a is preferably 1 or 2, and more preferably 2.

In the above general formula (Az-1), Base represents a nucleotide base. The nucleotide base is selected from the group consisting of guanine, cytosine, adenine, and uracil, as well as their derivatives. The above derivative is not particularly limited as long as the binding to the 3' end of RNA is not inhibited, and a known derivative of guanine, cytosine, adenine or uracil can be used. The nucleotide base is preferably selected from the group consisting of guanine, cytosine, adenine and uracil, and is more preferably cytosine.

Specific examples of the compound (Az-1) include pCp-azide.

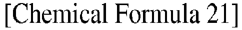

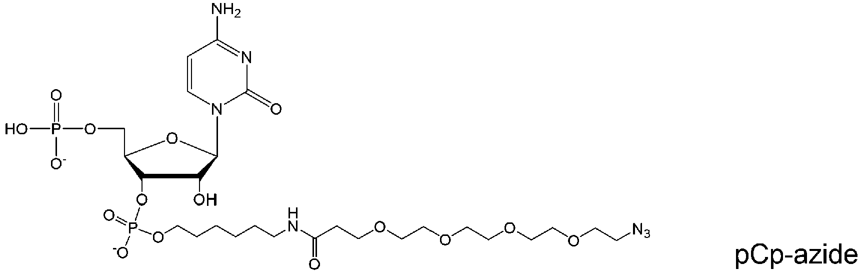

In the compound (1-pr), the above -OR¹C≡CH is preferably a propargyloxy group. Specific examples of the compound (1-pr) include Opp^{3'}GTP

Binding of the compound (Az-1) to the 3' end of RNA can be performed in the presence of T4 RNA ligase, in the same manner as in the above step (c1).The ligation reaction with T4 RNA ligase can be carried out in the same manner as in the above step (c1).

After the ligation reaction, RNA may be purified using a commercially available RNA purification kit or the like.

The step (c2) may be performed after the above step (a) as shown in the following Scheme (c2-1). Alternatively, as shown in the following Scheme (c2-2), it may be performed before the step (a). It should be noted that in the following Scheme, a case where the compound (1) is Opp^{3'}GTP is described as an example. In the following Scheme, a thick line (from 5' to 3') indicates an RNA strand.

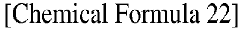

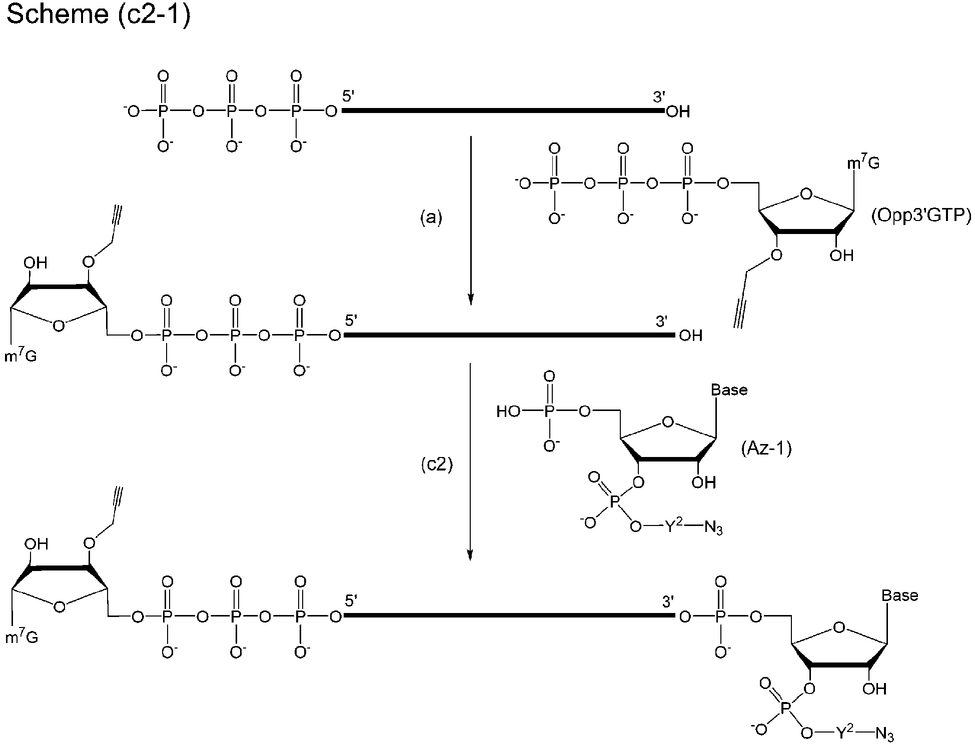

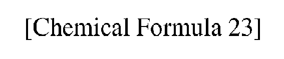

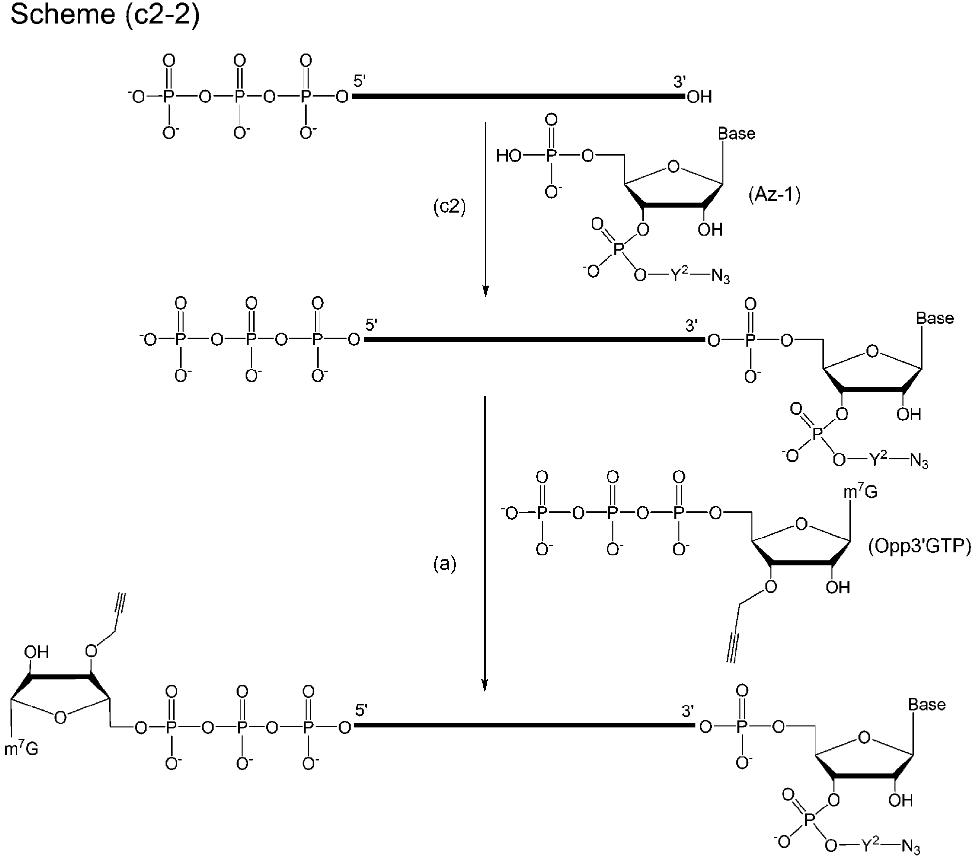

By carrying out the step (c2), an azide group can be introduced into the 3' end of RNA.

### (Step (d2))

The production method according to the present embodiment may further include, after the above steps (a) and (c2), a step (d2) of producing a modified circular RNA by reacting an alkynyl group introduced into the 5' end of RNA by the above step (a) with an azide group introduced into the 3' end of RNA by the above step (c2).

The reaction between the alkynyl group and the azide group can be carried out by the above-mentioned CuAAC reaction. The reaction conditions in the CuAAC reaction are not particularly limited, and the reaction conditions generally used for the CuAAC reaction can be used in the same manner as in the above step (d1).

A circular RNA can be obtained by binding the alkynyl group at the 5' end of RNA and the azide group at the 3' end by the CuAAC reaction.

The production method according to the present embodiment may further include a step of removing uncapped RNA, a step of purifying modified RNA, and the like.

In the method for producing a modified RNA according to the present embodiment, various modified RNAs in which various GTP analogs are introduced into the cap can be obtained by using a capping enzyme of vaccinia virus. As shown in the examples described later, since the vaccinia virus capping enzyme exhibits a wide range of substrate specificities for GTP analogs, various GTP analogs can be introduced into the 5' end of RNA. In the production method according to the present embodiment, it is not necessary to carry out complicated organic synthesis except for preparing a desired GTP analog, and various modified RNAs can be easily obtained.

The modified RNA obtained by the production method according to the present embodiment may have various modifications in the cap site. For this reason, modified RNAs having different degrees of stability and translational activity can be obtained. Therefore, a modified RNA having desired stability and translational activity can be selected and used as an expression vector. For example, it can be used as a gene therapy vector, a stem cell production vector, a gene editing vector, or the like.

Furthermore, by using click chemistry to introduce a functional group having a desired function, the desired function can be imparted to the modified RNA. For example, by introducing a fluorescent molecule such as AF647 into the modified RNA, the localization and dynamics of RNA in the cell can be observed, and it can also be used for identification of interacting molecules.

In addition, by using click chemistry to produce a circular RNA, resistance to exonucleases can be enhanced and stability in vivo can be improved.

Since the circular RNA produced by a conventional method does not have a 5' cap, it has been necessary to introduce an internal ribosome entry site (IRES) into the RNA sequence in order to initiate translation (for example, Wesselhoeft RA et al., Nat Commun. 2018 Jul 6; 9 (1): 2629.). The IRES may lose translational activity due to the use of a modified base (for example, _{Ψ} (pseudouracil), m¹_{Ψ}, m⁵C, or the like), and the use of a modified base may be restricted when the IRES is used.

On the other hand, since the circular RNA obtained by the production method according to the present embodiment has a 5' cap, translation is possible without introducing an internal ribosome entry site (IRES) into the RNA sequence. For this reason, various improvements such as improvement of stability by the introduction of a modified base can be made.

### [Modified RNA]

In one aspect, the present invention provides a modified RNA having a structure selected from the group consisting of the following formulas (Cp-1) to (Cp-13) at the 5' end. 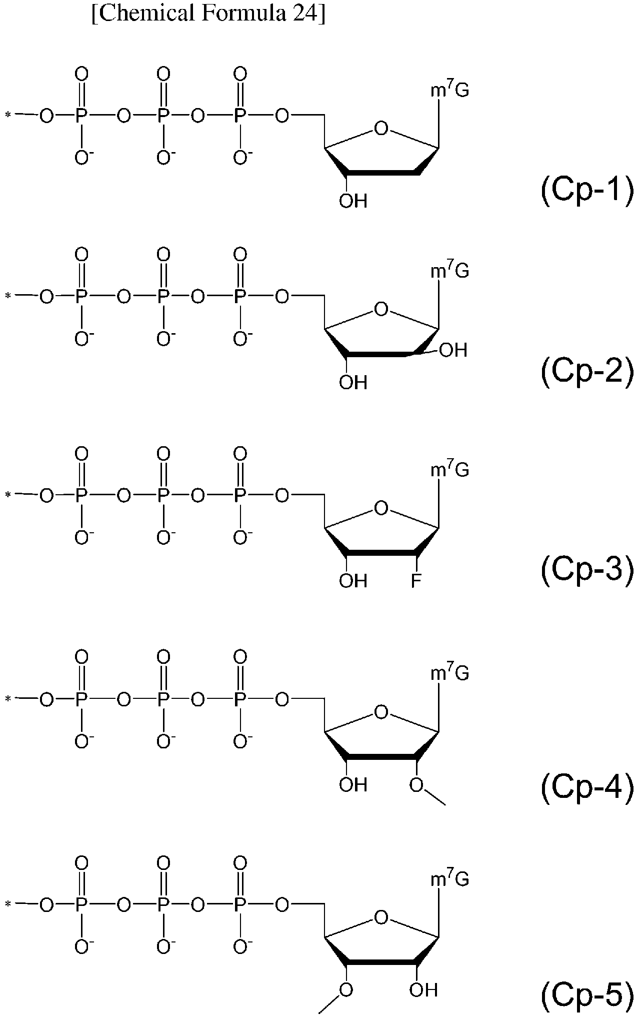 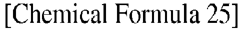 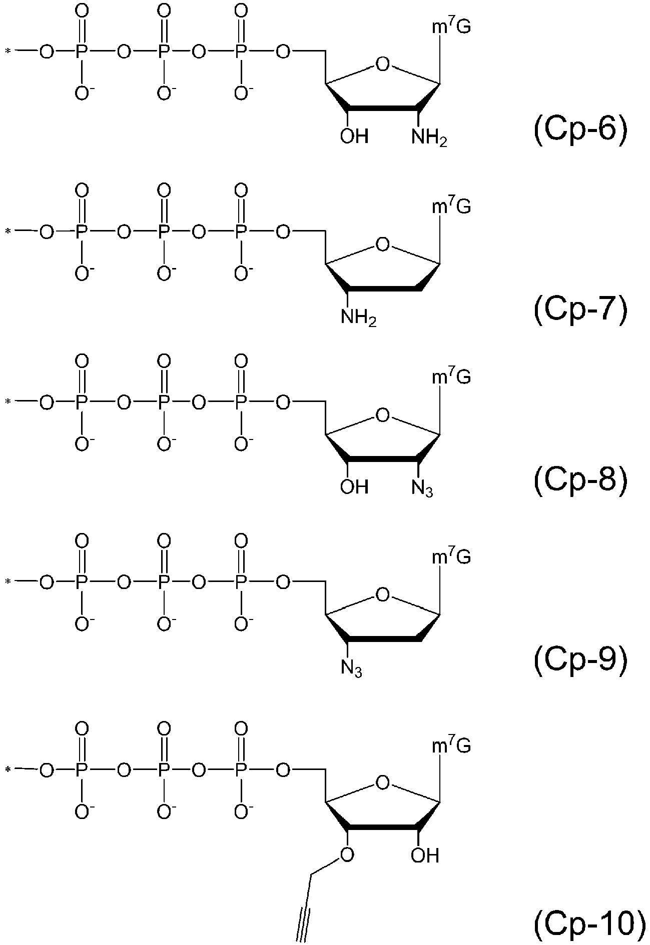 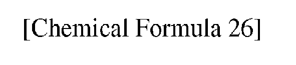 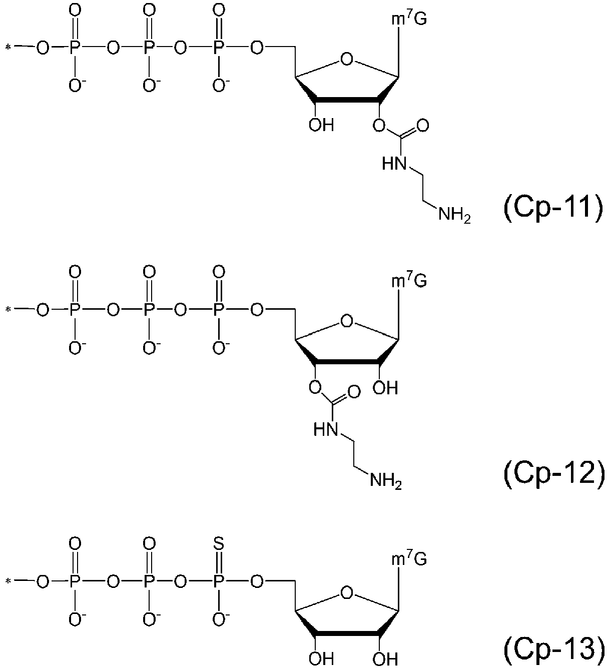 [In each formula, m⁷G represents N7-methylguanine; and ^{∗} represents a bond that binds to a carbon atom at a 5'position of an adjacent nucleoside.]

The modified RNAs (RNAs (Cp-1) to (Cp-13)) having the structures represented by the above formulas (Cp-1) to (Cp-13) respectively at the 5' ends can be produced by the method for producing a modified RNA of the above embodiment. RNAs (Cp-1) to (Cp-13) can be obtained by reacting dGTP, araGTP, fGTP, Om^{2'}GTP, Om^{3'}GTP, NH₂^{2'}CTP, NH₂^{3'}dGTP, N₃^{2'}GTP, N₃^{3'}dGTP, Opp^{3'}GTP, EDA^{2'}/^{3'}CTP (producing RNA (Cp-11) and RNA (Cp-12)), and GTPαS, respectively, with RNA in the presence of VCE. RNAs having these structures can be expected to improve stability. Further, an arbitrary molecule can be introduced by using a hydroxy group, an amino group, an azide group, a propargyl group or the like.

Among them, RNAs (Cp-8) to (Cp-10) can introduce a desired molecule by using click chemistry. For example, as in the above step (b1) or step (b2), modified RNAs having structures represented by the following general formulas (Ca-1) to (Ca-3), respectively, at the 5' ends can be obtained using click chemistry. Therefore, in one embodiment, the present invention also provides a modified RNA having a structure selected from the group consisting of the following general formulas (Ca-1) to (Ca-3) at the 5' end.

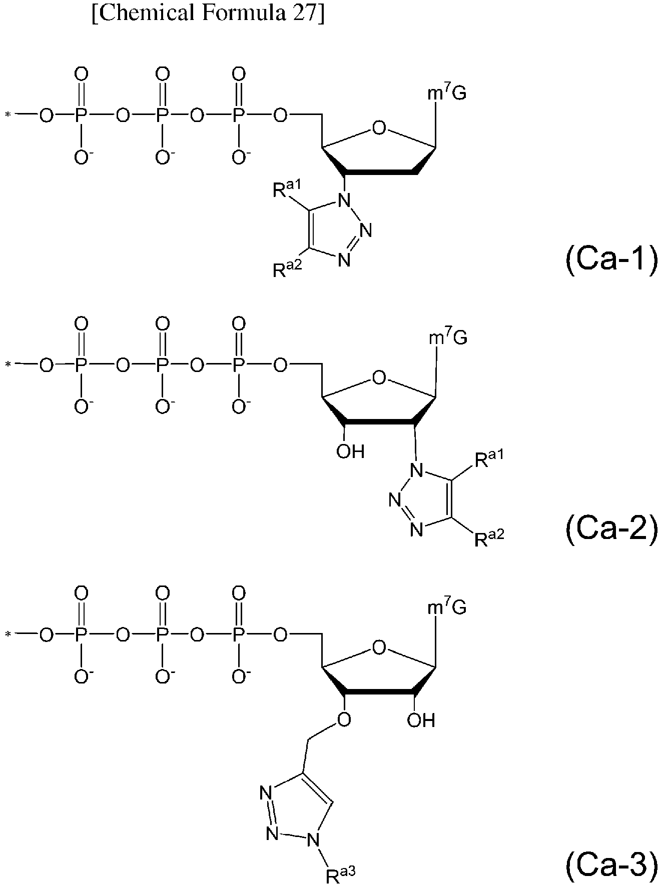

[In each formula, m⁷G represents N7-methylguanine; and ^{∗} represents a bond that binds to a carbon atom at a 5'position of an adjacent nucleotide residue; R^{a1} and R^{a2} each independently represent a hydrogen atom or an organic group, provided that at least one of R^{a1} and R^{a2} is an organic group. When both R^{a1} and R^{a2} are organic groups, they may bind with each other to form a ring structure. R^{a3} represents an organic group.]

In the above formula (Ca-1) or (Ca-2), R^{a1} and R^{a2} each independently represent a hydrogen atom or an organic group. The organic group is not particularly limited and can have a desired structure. In a preferred embodiment, either one of R^{a1} and R^{a2} is an organic group containing a functional group. Further, both R^{a1} and R^{a2} may be organic groups containing a functional group. The number of functional groups contained in the organic group is not particularly limited and can be a desired number. When R^{a1} and R^{a2} contain a plurality of functional groups, the plurality of functional groups may be the same as each other or may be different from each other. Examples of the functional group include the same as those exemplified in the above section entitled "[Method for producing modified RNA]".

R^{a1} and R^{a2} are not hydrogen atoms at the same time, and at least one of them is an organic group. Further, when both R^{a1} and R^{a2} are organic groups, R^{a1} and R^{a2} may bind with each other to form a ring structure.

In the above formula (Ca-3), R^{a3} is an organic group. The organic group is not particularly limited and can have a desired structure. In a preferred embodiment, R^{a3} is an organic group containing a functional group. The number of functional groups contained in R^{a3} is not particularly limited and can be a desired number. When R^{a3} contains a plurality of functional groups, the plurality of functional groups may be the same as each other or may be different from each other. Examples of the functional group include the same as those exemplified in the above section entitled "[Method for producing modified RNA]".

As a specific example of the structure represented by the above formula (Ca-1), for example, a structure represented by the following formula (Ca-1-1) is exemplified. Further, as a specific example of the structure represented by the above formula (Ca-2), for example, a structure represented by the following formula (Ca-2-1) is exemplified. Therefore, the present invention also provides a modified RNA having a structure selected from the group consisting of the following formulas (Ca-1-1) and (Ca-2-1) at the 5' end. 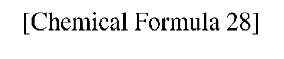 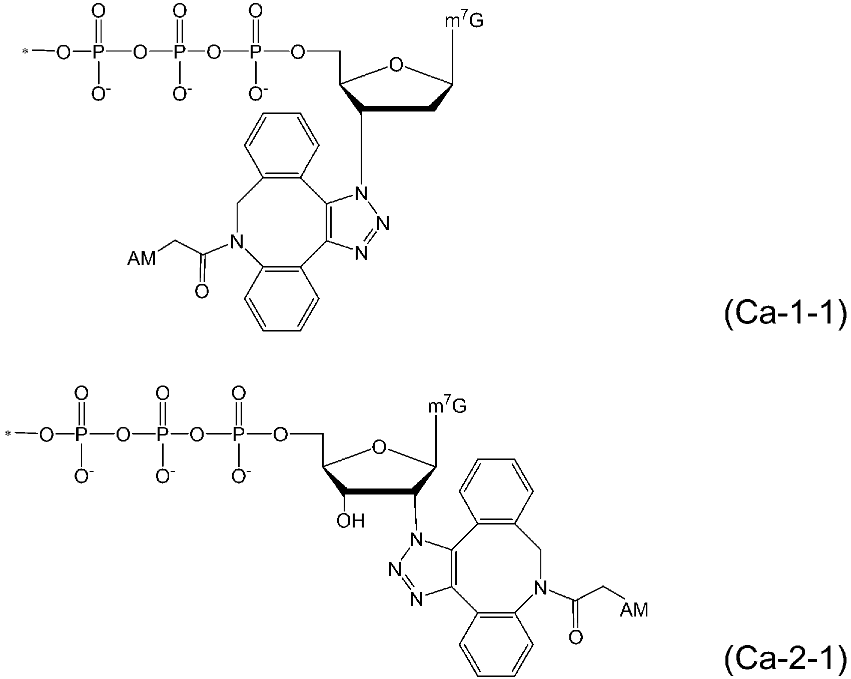 [In each formula, m⁷G represents N7-methylguanine; ^{∗} represents a bond that binds to a carbon atom at a 5'position of an adjacent nucleotide residue; and AM represents a functional group.]

Further, RNAs having structures represented by the formulas (Cp-8) to (Cp-10), respectively, at the 5' ends can be made into circular RNAs by using click chemistry. For example, as in the above steps (c1) and (d1) or the above steps (c2) and (d2), modified circular RNAs having structures represented by the following general formulas (Ci-1) to (Ci-3), respectively, can be obtained using click chemistry. Therefore, in one embodiment, the present invention also provides a modified circular RNA having a structure selected from the group consisting of the following general formulas (Ci-1) to (Ci-3).

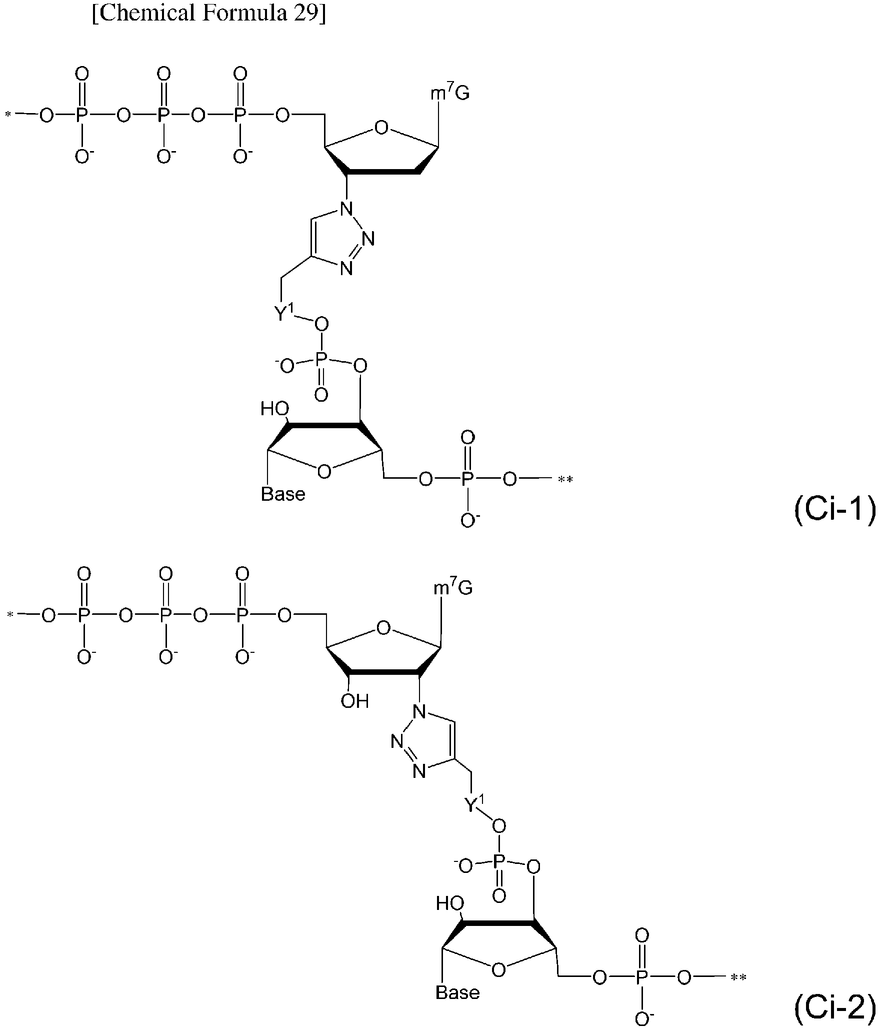

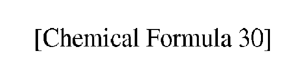

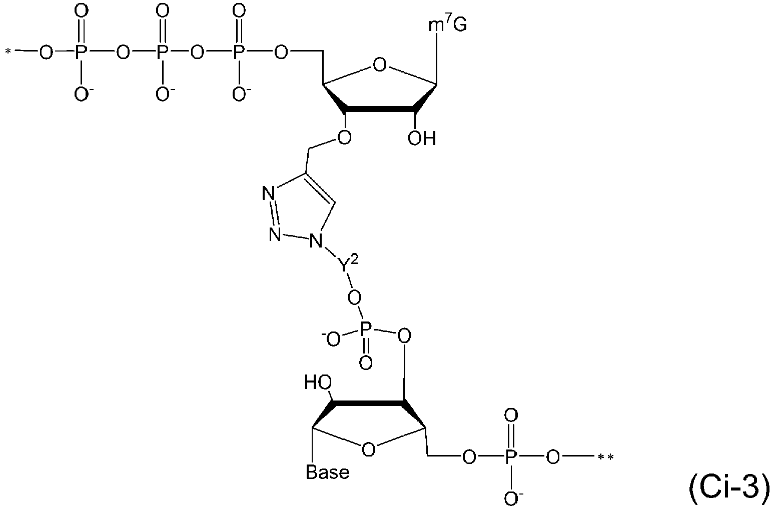

[In the formula, m⁷G represents N7-methylguanine; Y¹ and Y² each independently represent a divalent linking group; Base each independently represents a nucleotide base, ^{∗} represents a bond that binds to a carbon atom at a 5'position of an adjacent nucleotide residue, and ^{∗∗} represents a bond that binds to a carbon atom at a 3'position of an adjacent nucleotide residue.]

In the above formulas (Ci-1) and (Ci-2), Y¹ is the same as Y¹ defined in the above general formula (Ak-1), and the preferred examples thereof are also the same. In particular, preferred examples of Y¹ include ^{∗}1-(CH₂)ₘ-NHC(=O)-(CH₂O)ₙ-^{∗}2. In the above formula, ^{∗}1 is a bond that binds to an oxygen atom in the formula (Ci-1) or (Ci-2), and ^{∗}2 is a bond that binds to a carbon atom in a methylene group in the formula (Ci-1) or (Ci-2). Specific examples of Y¹ include ^{∗}1-(CH₂)₆-NHC(=O)-(CH₂O)5-^{∗}2.

Base each independently represents a nucleotide base, and is the same as Base defined in the above formula (Ak-1), and the preferred examples thereof are also the same. Specific examples of Base include cytosine.

In the above formula (Ci-3), Y² is the same as Y² defined in the above general formula (Az-1), and the preferred examples thereof are also the same. In particular, preferred examples of Y² include ^{∗}3-(CH₂)ₘ-NHC(=O)-(CH₂O)ₙ-(CH₂)₂-^{∗}4. In the above formula, ^{∗}3 is a bond that binds to an oxygen atom in the formula (Ci-3), and ^{∗}4 is a bond that binds to a nitrogen atom in the formula (Ci-3). Specific examples of Y² include ^{∗}3-(CH₂)₆-NHC(=O)-(CH₂O)₄-(CH₂)₂-^{∗}4.

Base each independently represents a nucleotide base, and is the same as Base defined in the above formula (Ak-1), and the preferred examples thereof are also the same. Specific examples of Base include cytosine.

The RNA according to the present embodiment can be used as a vector for gene therapy or stem cell production, or can be used for observing the intracellular dynamics of RNA, searching interacting molecules, and the like.

### [Examples]

Hereinafter, the present invention will be described with reference to Examples, but the present invention is not limited to the following Examples.

### [Materials]

### (Modified nucleotide)

m⁷GTP (Santa Cruz Biotechnology), dGTP (Toyobo), Opp^{3'}GTP, and EDA^{2'}/^{3'}GTP (Jena Bioscience) were used. Other GTP analogs were purchased from TriLink.

### (Enzyme)

As a capping enzyme derived from Vaccinia virus (VCE), the ScriptCap m⁷G Capping System (CellScript) was used.

### [Example 1] Evaluation of capping efficiency of GTP analog

### <Method>

Using various GTP analogs, RNA capping reactions by VCE were carried out, and the capping efficiencies of GTP analogs were evaluated. In order to clearly detect the cap addition to RNA, 11 nucleotide (nt) long short strand RNA (5'-GGGCGAAUUAA-3' (SEQ ID NO: 1): the same sequence as that of the 5' end of the 5'UTR of a reporter mRNA) was synthesized and used. A double stranded DNA having a T7 promoter sequence upstream of the target sequence was prepared as a transcription template, and a transcription reaction was carried out overnight using a MEGAshortscript T7 Transcription Kit (Thermo Fisher Scientific). The transcription reaction was carried out at 42°C in order to suppress the addition of extra nucleotides to the 3' end of RNA that occurs during transcription. After the transcription reaction, denatured polyacrylamide gel electrophoresis (denatured PAGE) of a transcription reaction product was performed to cut out and purify an RNA of a target size.

The above purified short strand RNA was subjected to a capping reaction using VCE. 200 pmol of RNA was reacted using 20 nmol of a GTP analog and 8 units of VCE at 37°C for 1 hour or 24 hours. After the capping reaction, denatured PAGE of a capping reaction product was performed, and after staining with SYBR Green II (Lonza), the gel was observed with Typhoon FLA 7000 (GE Healthcare). The capping efficiency was calculated from the band intensity ratio of capped RNA to uncapped RNA using Image Quant (GE Healthcare). Each experiment was performed 3 times or more, and the average value and the standard deviation were calculated.

### <Results>

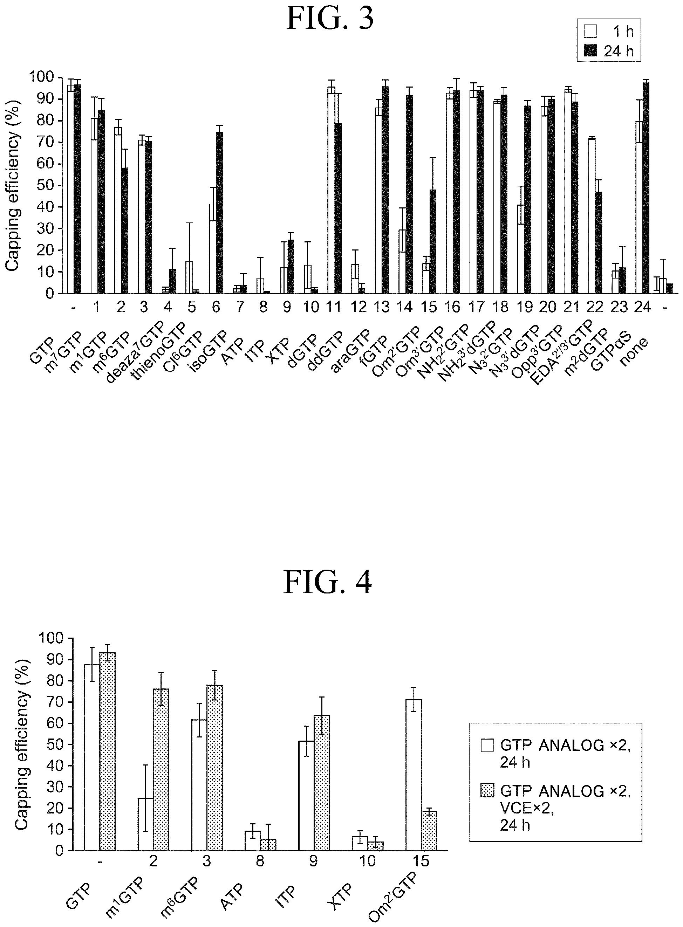

The result of confirming the capping efficiency of the GTP analogs described in FIG. 2 is shown in FIG. 3. The capping efficiency of each GTP analog was calculated from the amount of RNA to which the GTP analog was added and the amount of unreacted RNA. Capping efficiencies equivalent to that of GTP were confirmed with a plurality of GTP analogs (for example, dGTP (No. 11), Om^{3'}GTP (No. 16), and the like).

Further, for the GTP analogs having low capping efficiencies in FIG. 3, the capping efficiencies when the concentrations of the GTP analog and VCE in the capping reaction were changed are shown in FIG. 4. With respect to Om^{2'}GTP (No. 15), the capping efficiency was greatly improved by doubling the concentration of the GTP analog. Further, by doubling the VCE concentration together with that of the GTP analog, the capping efficiencies of m¹GTP (No. 2), m⁶GTP (No. 3), and 1TP (No. 9) were improved.

The results of FIGS. 3 and 4 are summarized in Table 1. In Table 1, "A" indicates that capping was observed, and "B" indicates that either capping was not observed, or the capping efficiency was low.

**[Table 1]**

| No. | Abbreviation | Compound name | Capping efficiency |
|---|---|---|---|
| 1 | m⁷GTP | N7-Methylguanosine-5'-triphosphate | A |
| 2 | m¹GTP | N1-Methylguanosine-5'-triphosphate | A |
| 3 | m⁶GTP | O6-Methylguanosine-5'-triphosphate | A |
| 4 | deaza⁷GTP | 7-Deazaguanosine-5'-Triphosphate | B |
| 5 | thienoGTP | Thienoguanosine-5' -Triphosphate | B |
| 6 | C1⁶GTP | 6-Chloroguanosine-5'-Triphosphate | A |
| 7 | isoGTP | Isoguanosine-5'-triphosphate | B |
| 8 | ATP | Adenosine-5' -triphosphate | B |
| 9 | 1TP | lnosine-5'-triphosphate | A |
| 10 | XTP | Xanthosine-5' -Triphosphate | B |
| 11 | dGTP | 2'-Deoxyguanosine-5'-triphosphate | A |
| 12 | ddGTP | 2',3'-Dideoxyguanosine-5'-triphosphate | B |
| 13 | araGTP | Araguanosine-5'-triphosphate | A |
| 14 | fGTP | 2'-Fluoro-2'-deoxvguanosine-5'-Triphosphate | A |
| 15 | Om²'GTP | 2'-O-Methylguanosine-5' -Triphosphate | A |
| 16 | Om³' GTP | 3'-O-Methylguanosine-5'-Triphosphate | A |
| 17 | NH₂ ²'GTP | 2'-Amino-2'-deoxyguanosine-5'-Triphosphate | A |
| 18 | NH₂³'dGTP | 3'-Amino-2',3'-dideoxyguanosine-5'-Triphosphate | A |
| 19 | N₃²'GTP | 2' -Azido-2'-deoxyguanosine-5'-Triphosphate | A |
| 20 | N₃^{3'}dGTP | 3'-Azido-2',3'-dideoxyguanosine-5'-Triphosphate | A |
| 21 | Opp^{3'}GTP | 3'-(O-Propargyl)-GTP | A |
| 22 | EDA^{2'/3'}GTP | 2'/3'-O-(2-Aminoethyl-carbamoyl)-Guanosine-5'-triphosphate | A |
| 24 | GTPαS | Guanosine- 5'-O-(1-Thiotriphosphate) | A |

### [Example 2] Evaluation of intracellular translational activity of cap-modified mRNA

### <Method>

### (Production of cap-modified mRNA)

PCR using KOD-Plus-Neo (TOYOBO) was performed using a plasmid encoding a fluorescent protein Azami-Green (hmAG1) as a template, and a DNA fragment for a template for mRNA transcription was amplified. Subsequently, the amplified product was purified using a QIAquick PCR Purification Kit (Qiagen). Using the above amplified product as a transcription template, a transcription reaction was carried out at 37°C for 6 hours using a MEGAscript T7 Transcription Kit (Thermo Fisher Scientific). For the above transcription reaction, 5-Methyl-CTP (TriLink) and Pseudo-UTP (TriLink) were used, instead of CTP and UTP, respectively, in order to suppress the immune response that occurs when RNA was introduced into cells. The mRNA obtained by the above transcription reaction was column-purified using an RNeasy MinElute Cleanup Kit (Qiagen) after a DNase treatment.

Capping of mRNA by a GTP analog was carried out using VCE. 50 pmol of mRNA was reacted using 20 nmol of a GTP analog and 8 units of VCE at 37°C for 1 hour or 24 hours. In the capping reaction with a GTP analog having a low capping efficiency, the amounts of the GTP analog and VCE were increased to twice the above amounts.

After the capping reaction, the uncapped mRNA was degraded and removed. Removal of the uncapped mRNA was performed as follows. The mRNA on which the capping reaction had been conducted was dephosphorylated by Antarctic phosphatase (New England Biolabs) and phosphorylated by T4 Polynucleotide kinase (Takara), and the 5' end of the uncapped mRNA was monophosphorylated. Subsequently, using the Terminator 5'-Phosphate-Dependent Exonuclease (Epicentre), which was an enzyme that specifically degrades only monophosphorylated RNA, a degradation reaction of uncapped mRNA was carried out at 30°C for 1 hour. As a result, the cap-modified mRNA was purified.

iRFP670 mRNA was used as a transfection control. A transcription template for iRFP670 mRNA was produced in the same manner as that described for hmAG1 mRNA. Using the above iRFP670 mRNA transcription template, a mixture of an anti-reverse cap analog (ARCA: TriLink) and GTP (4: 1) was added instead of GTP to perform a transcription reaction. In the transcription reaction in the presence of ARCA, ARCA is not incorporated into the 5' end and RNAs having 5' triphosphates are also produced. In order to prevent the immune response and cell death derived from the above 5'phosphate group, the transcript was dephosphorylated and then used for transfection.

### (Evaluation of translational activity in human cells)

HeLa cells or HEK293FT cells were seeded on a 24-well plate 24 hours before transfection. HeLa cells were seeded to a density of 0.5 × 10⁵ cells/well, and HEK293FT cells were seeded to a density of 1.0 × 10⁵ cells/well. The modified capped hmAG1 mRNA (100 ng) that had been produced was transfected together with iRFP670 mRNA (100 ng) using Lipofectamine MessengerMAX (Invitrogen). Medium exchange was performed 4 hours after transfection, and the expression level of the fluorescent protein was examined 24 hours after transfection using a fluorescence microscope (IX81: Olympus) and a flow cytometer (BD Accuri C6: BD Biosciences). The measurement results by the flow cytometer were analyzed using FlowJo (BD Biosciences). The expression level of Azami-Green was standardized by the expression level of cotransfected iRFP670. Each experiment was performed 3 times.

### <Results>

Chemical modifications of the 5' end cap may affect the translation initiation and mRNA stability, and change the translational activity. Accordingly, for the GTP analogs whose high capping efficiencies had been confirmed in Example 1, mRNAs having 5' end caps by the GTP analogs were produced and transfected into human cells to examine the translational activity in human cells.

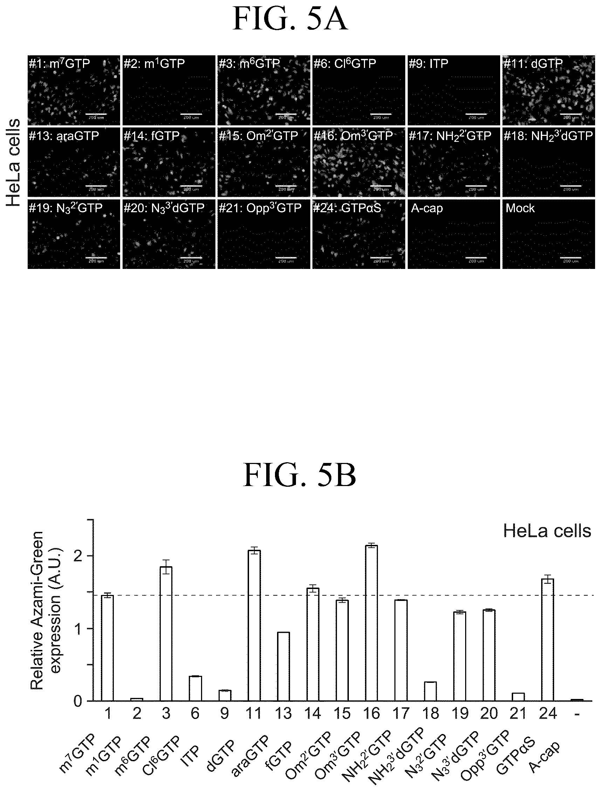

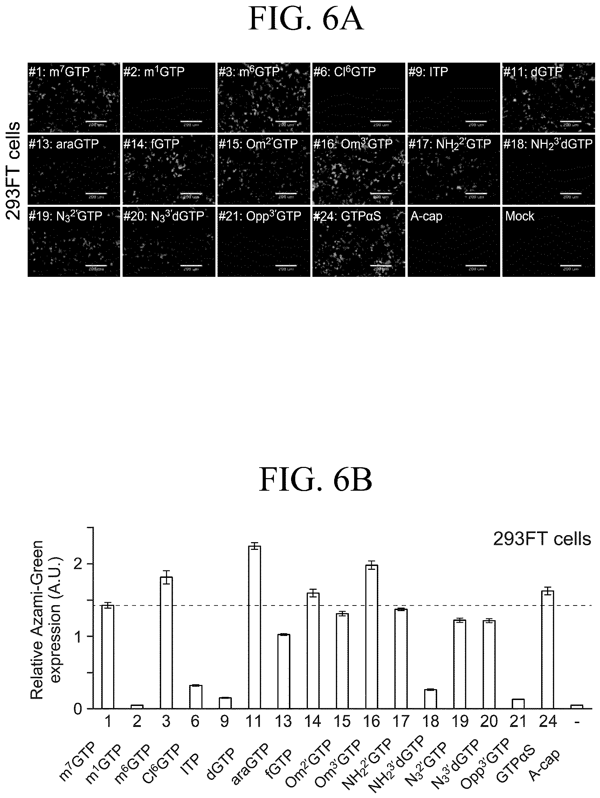

The results are shown in FIGS. 5A and 5B (HeLa cells) and FIGS. 6A and 6B (293FT cells). FIGS. 5A and 6A are observation results by the fluorescence microscope, and FIGS. 5B and 6B are measurement results by the flow cytometer. Since it was confirmed that the capping efficiency of m⁷GTP (No. 1) which had the same cap structure as the cap structure by GTP was favorable in Example 1, the mRNA capped with m⁷GTP was used as an RNA standard.

All the results for HeLa cells and 293FT cells showed the same trend. m⁶GTP (No. 3), dGTP (No. 11), fGTP (No. 14), Om^{3'}GTP (No. 16) and GTPαS (No. 24) showed higher translational activities than that of m⁷GTP (No. 1). Om^{2'}GTP (No. 15) and NH₂^{2'}GTP (No. 17) showed the same level of translational activity as that of m⁷GTP (No. 1). Although the translational activities were slightly reduced with araGTP (No. 13), N₃^{2'}GTP (No. 19), and N₃^{3'}dGTP (No. 20), the extent of the reduction was small. The translational activities were reduced with m¹GTP (No. 2), C1⁶GTP (No. 6), ITP (No. 9), NH₂^{3'}dGTP (No. 18), and Opp^{3'}GTP (No. 21).

From the above results, it was confirmed that RNAs having various translational activities can be produced by adding caps with various structures to the 5' ends of the RNAs using VCE and various GTP analogs.

### [Example 3] Modification of 5' cap

### <Method>

### (Bioconjugation)

The short strand RNA produced in Example 1 was subjected to a capping reaction by VCE using GTP, N₃^{2'}GTP, or N₃^{3'}dGTP. The capping reaction was carried out in the same manner as in Example 1 except that the above-mentioned GTP analogs were used. After the capping reaction, denatured PAGE was performed, and a capped RNA fraction was cut out from the gel and purified. 200 pmol of the obtained RNA was mixed with 100 nmol of DBCO-biotin (Dibenzocyclooctyne-PEG4-biotin conjugate, Aldrich) or DBCO-AF647 (AF 647 DBCO, Click Chemistry Tools), and reacted at 37°C for 1 hour in a reaction solution containing 1 × Tris-buffered saline (TBS: 50 mM Tris, 150 mM NaCl, pH 7.6) and 25% dimethyl sulfoxide (DMSO). After the reaction, unreacted DBCO was removed by ethanol precipitation, followed by denatured PAGE and observation with Typhoon FLA 7000.

### (Visualization of fluorescently labeled mRNA in HeLa cells)

The hmAG1 mRNA produced in Example 2 was subjected to a capping reaction by VCE using GTP, N₃^{2'}GTP, or N₃^{3'}dGTP, and a degradation treatment of uncapped RNA. The capping reaction and the degradation treatment of uncapped RNA were carried out in the same manner as in Example 2 except that the above-mentioned GTP analogs were used. Approximately 11 pmol of the above mRNA was mixed with 100 nmol of DBCO-AF647, and reacted at 37°C for 1 hour in a reaction solution containing 1 × Tris-buffered saline (TBS: 50 mM Tris, 150 mM NaCl, pH 7.6) and 25% DMSO. After the reaction, column purification of the reaction solution was performed, followed by denatured PAGE to confirm that the mRNA was fluorescently labeled and that no unreacted dye was brought in.

24 hours before transfection, HeLa cells were seeded on a glass bottom 24-well plate (Greiner) to a density of 0.5 × 10⁵ cells/well. The above cells were transfected with 500 ng of hmAG1 mRNA using Lipofectamine MessengerMAX. 4 hours after transfection, cells were washed with PBS and treated with a 1% paraformaldehyde solution for 20 minutes for fixation. Then, they were stained with Hoechst 33342 (Thermo Fisher Scientific) and observed with a confocal microscope (LSM710, Carl Zeiss).

### <Results>

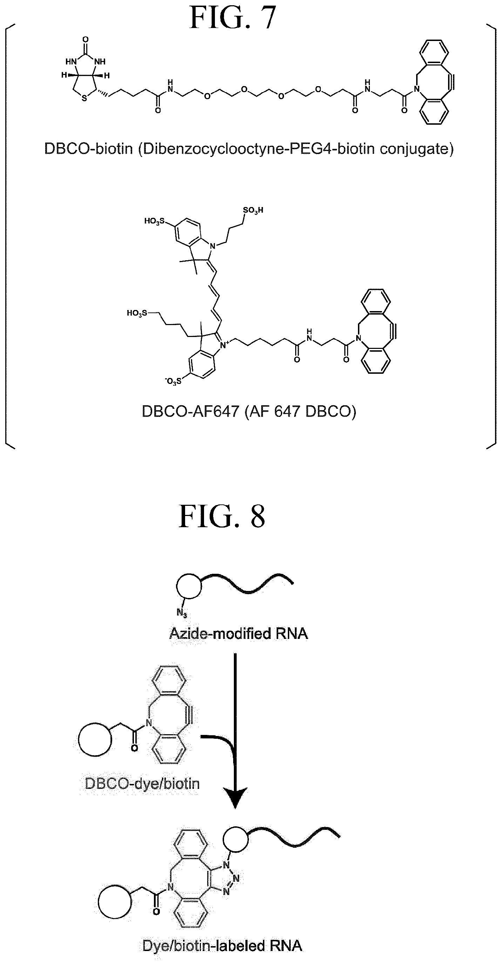

Among the GTP analogs that could be used for capping by VCE, those having a primary amino group, an azide group, and a propargyl group (alkyne) were included. These functional groups have been widely used in bioconjugation in which functional molecules are covalently linked to biomolecules such as proteins and nucleic acids. For this reason, it is considered that the 5' end cap having these functional groups can be further modified by bioconjugation techniques. Accordingly, an attempt has been made to introduce a functional molecule into an azide group in the cap by using the reaction between azide and alkyne, which is a typical type of click chemistry. Using two types of functional molecules (DBCO-biotin, DBCO-AF647: see FIG. 7) containing DBCO (Dibenzocyclooctyne) as an alkyne, a capped RNA having an azide group was modified using the strain-promoted azide-alkyne cycloaddition (SPAAC) reaction (see FIG. 8).

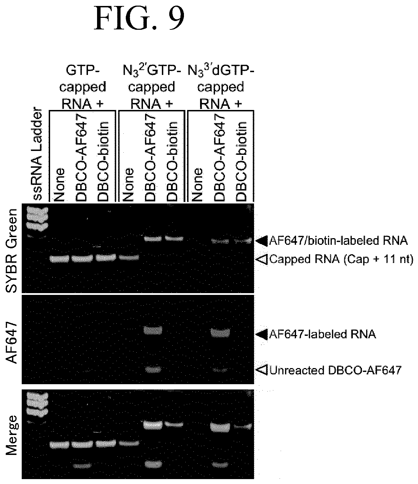

The results are shown in FIG. 9. It was possible to specifically add biotin or a fluorescent dye to the capped RNA having an azide group.

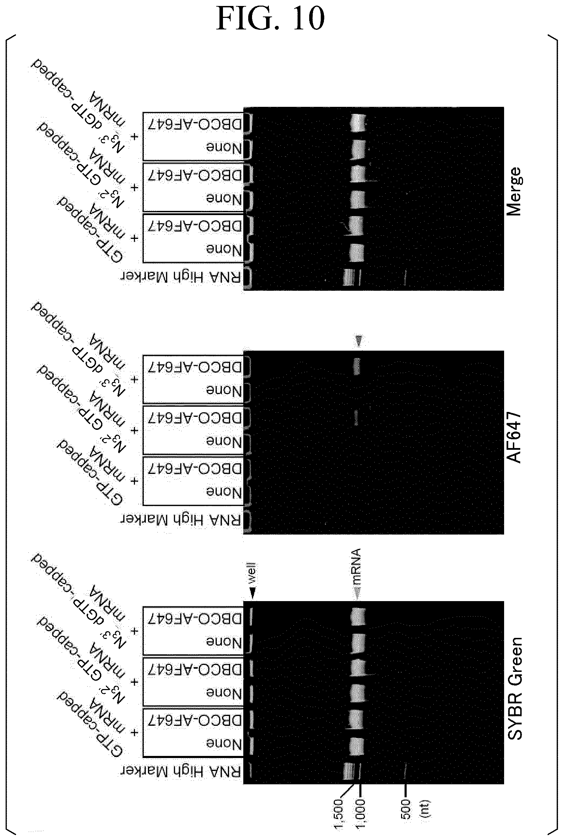

Next, the fluorescent dye AF647 was introduced into the capped mRNA having an azide group using the SPAAC reaction. As shown in FIG. 10, it was confirmed that AF647 was introduced (labeled with AF647) specifically into the capped RNA having an azide group.

Next, the AF647-labeled mRNA or AF647-unlabeled mRNA was transfected into HeLa cells, and it was confirmed whether the introduced mRNA could be detected intracellularly.

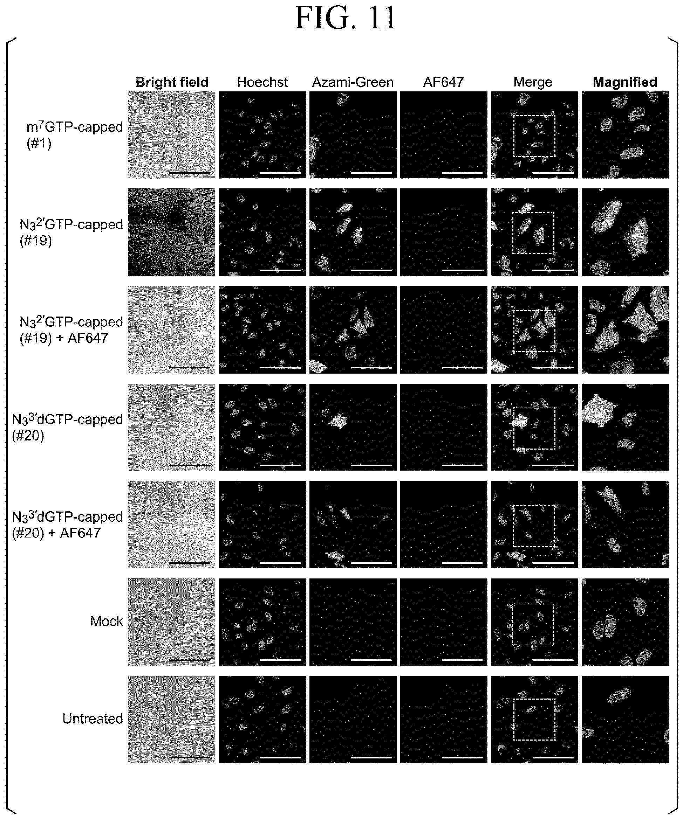

The results are shown in FIG. 11. FIG. 11 is a confocal microscope image of cells into which AF647-labeled mRNA or AF647-unlabeled mRNA has been introduced. In the cells into which AF647-labeled mRNA had been introduced, the red fluorescence of AF647 present in the form of granules in the cytoplasm could be detected.

From the above results, it was shown that various functional molecules can be introduced into the RNA cap site by combining capping by VCE using GTP analogs with bioconjugation techniques.

### [Example 4] Production of circular RNA

### <Method>

The hmAG1 mRNA was synthesized by an in vitro transcription method in the same manner as in Example 2, followed by column purification. 100 pmol of the above mRNA was reacted at 16°C for 48 hours in a reaction solution containing 200 pmol of pCp-alkyne (Jena Bioscience), 20 units of T4 RNA Ligase (Ambion), and 500 pmol of ATP. The reaction product was purified with the RNeasy MinElute Cleanup Kit (Qiagen). The 5' end of the RNA obtained by purification was capped with N₃^{3'}dGTP under the same conditions as in Example 2, and purified using the RNeasy MinElute Cleanup Kit (Qiagen). 10 pmol of the obtained RNA was reacted at 25°C for 1 hour in a reaction solution containing 0.1 mM CuSO₄, 0.5 mM BTTAA (Click Chemistry Tools), 25 mM phosphate buffer, 20% DMS, and 0.5 mM ascorbic acid. Then, the resulting reaction product was purified by ethanol precipitation, and the obtained RNA was confirmed by denatured PAGE.

### <Results>

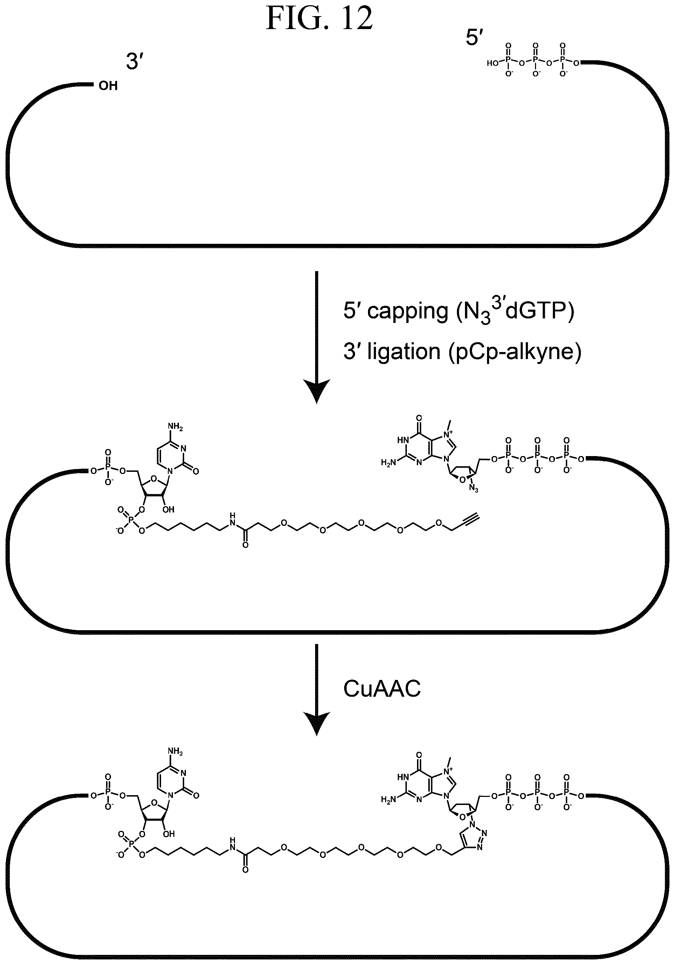

It is considered that a circularized RNA can be obtained as shown in FIG. 12 by carrying out the CuAAC reaction with respect to the RNA capped with N3^{3'}dGTP and introduced with pCp-alkyne at the 3' end.

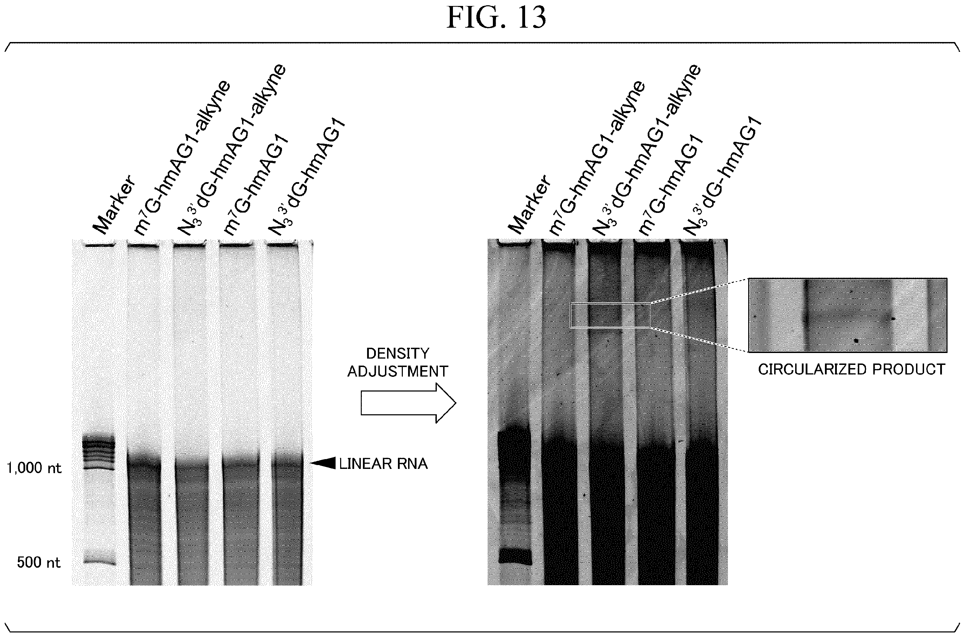

As a result of denatured PAGE, a low mobility band was confirmed in electrophoresis with respect to the RNA (N₃^{3'}dG-hmAG1-alkyne) capped with N₃^{3'}dGTP and introduced with pCp-alkyne at the 3' end (see FIG. 13). On the other hand, no such band could be observed with respect to the RNA having a natural cap (m⁷G-hmAG1), the RNA obtained by introducing pCp-alkyne at the 3' end of RNA having a natural cap (m⁷G-hmAG1-alkyne), and the RNA capped with N₃^{3'}dGTP with no introduction of pCp-alkyne at the 3' end (N₃^{3'}dG-hmAG1). From these results, it was considered that a circular RNA was produced with respect to the RNA capped with N₃^{3'}dGTP and introduced with pCp-alkyne at the 3' end.

### [Industrial Applicability]

According to the present invention, there are provided an RNA capping method capable of easily introducing various modifications into a 5' cap site, a method for producing a modified RNA using the aforementioned capping method, and a novel modified RNA produced by the aforementioned method for producing a modified RNA.

## Claims

1. An RNA capping method comprising
a step of reacting a compound (1) represented by the following general formula (1), provided that GTP is excluded, with an RNA in the presence of a Vaccinia virus capping enzyme, 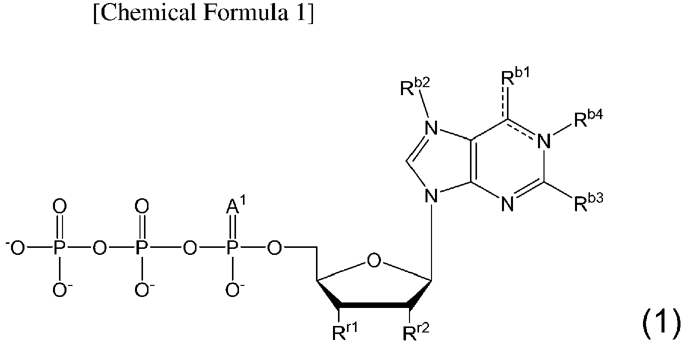
wherein R^{b1} represents an oxo group, an alkoxy group having 1 to 3 carbon atoms, or a halogen atom; R^{b2} is either absent or represents an alkyl group having 1 to 3 carbon atoms; R^{b3} represents an amino group or a hydrogen atom; R^{b4} is either absent or represents a hydrogen atom or an alkyl group having 1 to 3 carbon atoms; R^{r1} represents a hydroxy group, an alkoxy group having 1 to 3 carbon atoms, an amino group, an azide group, -OR¹C≡CH, or -R²R³; R^{r2} represents a hydrogen atom, a hydroxy group, a halogen atom, an alkoxy group having 1 to 3 carbon atoms, an amino group, an azide group, -OR¹C≡CH, or -R²R³; A¹ represents an oxygen atom or a sulfur atom;
R¹ represents an alkylene group having 1 to 3 carbon atoms, R² represents - OC(=O)NH-, -OC(=O)-, -NHC(=O)-, -O- or a single bond; and R³ represents an aminoalkyl group having 1 to 3 carbon atoms; and
a double line composed of a solid line and a dotted line represents a single bond or a double bond.

2. The RNA capping method according to Claim 1,
wherein the compound (1) is selected from the group consisting of N7-methylguanosine-5' -triphosphate, N1 -methylguanosine-5' -triphosphate, 06-methylguanosine-5' -triphosphate, 6-chloroguanosine-5' -triphosphate, inosine-5'-triphosphate, 2'-deoxyguanosine-5'-triphosphate, araguanosine-5'-triphosphate, 2'-fluoro-2'-deoxyguanosine-5'-triphosphate, 2'-O-methylguanosine-5'-triphosphate, 3'-0-methylguanosine-5' -triphosphate, 2' -amino-2' -deoxyguanosine-5' -triphosphate, 3'-amino-2',3'-dideoxyguanosine-5' -triphosphate, 2' -azido-2' -deoxyguanosine-5'-triphosphate, 3'-azido-2',3'-dideoxyguanosine-5'-triphosphate, 3'-(O-propargyl)-guanosine-5'-triphosphate, 2'/3'-O-(2-aminoethyl-carbamoyl)-guanosine-5'-triphosphate and guanosine-5' -O-(1 -thiotriphosphate).

3. A method for producing a modified RNA, comprising a step (a) of reacting a compound (1) represented by the following general formula (1), provided that GTP is excluded, with an RNA in the presence of a Vaccinia virus capping enzyme, 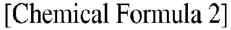 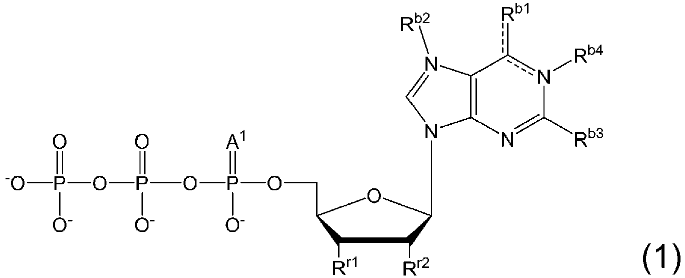
wherein R^{b1} represents an oxo group, an alkoxy group having 1 to 3 carbon atoms, or a halogen atom; R^{b2} is either absent or represents an alkyl group having 1 to 3 carbon atoms; R^{h3} represents an amino group or a hydrogen atom; R^{b4} is either absent or represents a hydrogen atom or an alkyl group having 1 to 3 carbon atoms; R^{r1} represents a hydroxy group, an alkoxy group having 1 to 3 carbon atoms, an amino group, an azide group, -OR¹C≡CH, or -R²R³; R^{r2} represents a hydrogen atom, a hydroxy group, a halogen atom, an alkoxy group having 1 to 3 carbon atoms, an amino group, an azide group, -OR¹C≡CH, or -R²R³; A¹ represents an oxygen atom or a sulfur atom;
R¹ represents an alkylene group having 1 to 3 carbon atoms, R² represents - 0C(=O)NH-, -OC(=O)-, -NHC(=O)-, -O- or a single bond; and R³ represents an aminoalkyl group having 1 to 3 carbon atoms; and
a double line composed of a solid line and a dotted line represents a single bond or a double bond.

4. The method for producing a modified RNA according to Claim 3,
wherein the compound (1) is selected from the group consisting of N7-methylguanosine-5'-triphosphate, N1-methylguanosne-5' -triphosphate, O6-methylguanosine-5' -triphosphate, 6-chloroguanosine-5' -triphosphate, inosine-5'-triphosphate, 2'-deoxyguanosine-5'-triphosphate, araguanosine-5'-triphosphate, 2'-fluoro-2'-deoxyguanosine-5' -triphosphate, 2' -O-methylguanosine-5'-triphosphate, 3'-O-methylguanosine-5' -triphosphate, 2'-amino-2'-deoxyguanosine-5'-triphosphate,3'-amino-2',3'-dideoxyguanosine-5' -triphosphate, 2' -azido-2' -deoxyguanosine-5'-triphosphate, 3'-azido-2',3'-dideoxyguanosine-5'-triphosphate, 3'-(O-propargyl)-guanosine-5'-triphosphate, 2'/3'-O-(2-aminoethyl-carbamoyl)-guanosine-5'-tuiphosphate and guanosine-5' -O-(1-thiotriphosphate).

5. The method for producing a modified RNA according to Claim 3, further comprising, after the step (a),
a step (bl) of reacting an alkyne compound,
wherein R^{r1} or R^{r2} in the general formula (1) is an azide group.

6. The method for producing a modified RNA according to Claim 5, wherein the compound (1) is 2'-azido-2'-deoxyguanosine-5'-triphosphate or 3'-azido-2',3'-dideoxyguanosine-5' -triphosphate.

7. The method for producing a modified RNA according to Claim 5 or 6, wherein the alkyne compound comprises a functional group.

8. The method for producing a modified RNA according to Claim 3, further comprising, after the step (a),
a step (b2) of reacting an azide compound,
wherein R^{r1} or R^{r2} in the general formula (1) is -OR¹C≡CH.

9. The method for producing a modified RNA according to Claim 8, wherein the compound (1) is 3'-(O-propargyl)-guanosine-5'-triphosphate.

10. The method for producing a modified RNA according to Claim 8 or 9, wherein the azide compound comprises a functional group.

11. The method for producing a modified RNA according to Claim 3, further comprising
a step (cl) of binding a compound (Ak-1) represented by the following general formula (Ak-1) to a 3' end of the RNA,
wherein R^{r1} or R^{r2} in the general formula (1) is an azide group, 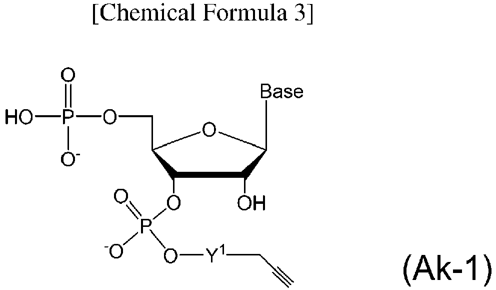
wherein Y¹ represents a divalent linking group; and Base represents a nucleotide base.

12. The method for producing a modified RNA according to Claim 11, further comprising, after the step (a) and the step (cl),
a step (dl) of producing a circular RNA by reacting an azide group introduced into a 5' end of the RNA by the step (a) with an alkynyl group introduced into a 3' end of the RNA by the step (cl).

13. The method for producing a modified RNA according to Claim 11 or 12, wherein the compound (1) is 2'-azido-2'-deoxyguanosine-5'-triphosphate or 3'-azido-2' ,3' -dideoxyguanosine-5' -triphosphate.

14. The method for producing a modified RNA according to Claim 3, further comprising
a step (c2) of binding a compound (Az-1) represented by the following general formula (Az-1) to a 3' end of the RNA,
wherein R^{r1} or R^{r2} in the general formula (1) is -OR¹C≡CH, 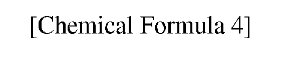 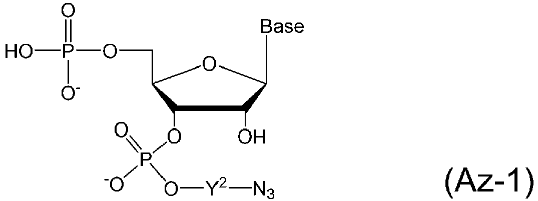
wherein Y² represents a divalent linking group; and Base represents a nucleotide base.

15. The method for producing a modified RNA according to Claim 14, further comprising, after the step (a) and the step (c2),
a step (d2) of producing a circular RNA by reacting an alkynyl group introduced into a 5' end of the RNA by the step (a) with an azide group introduced into a 3' end of the RNA by the step (c2).

16. The method for producing a modified RNA according to Claim 14 or 15, wherein the compound (1) is 3'-(O-propargyl)-guanosine-5'-triphosphate.

17. A modified RNA comprising a structure selected from the group consisting of the following formulas (Cp-1) to (Cp-13) at a 5' end, 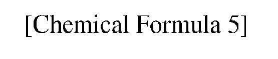 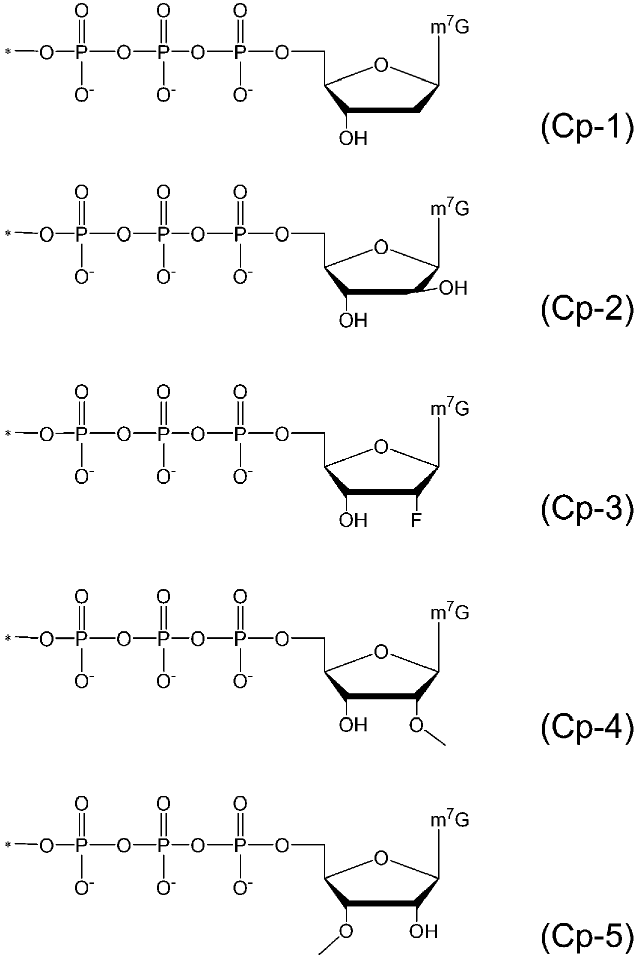 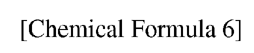 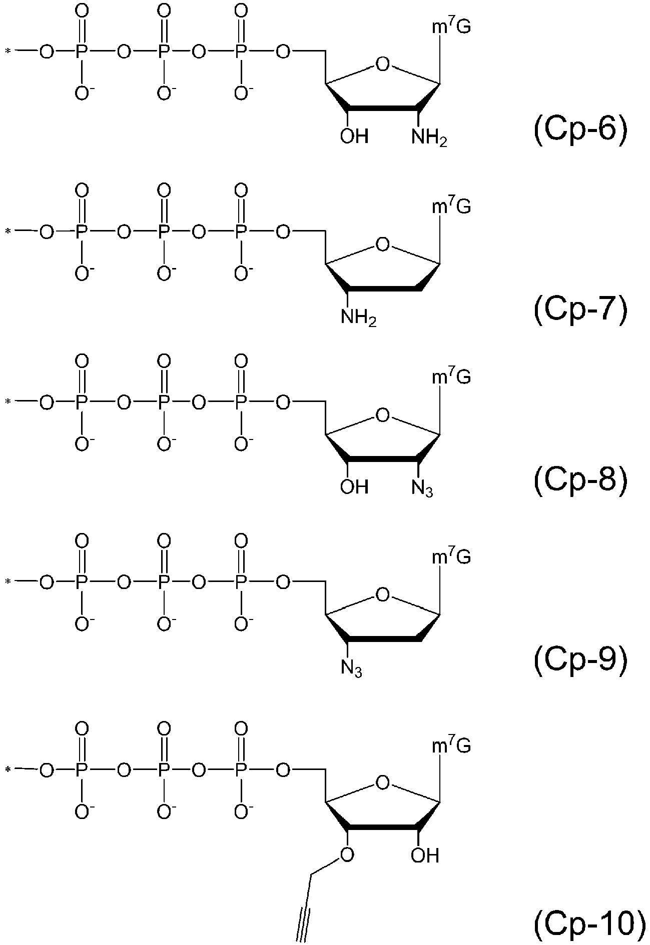 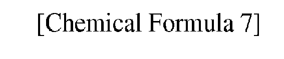 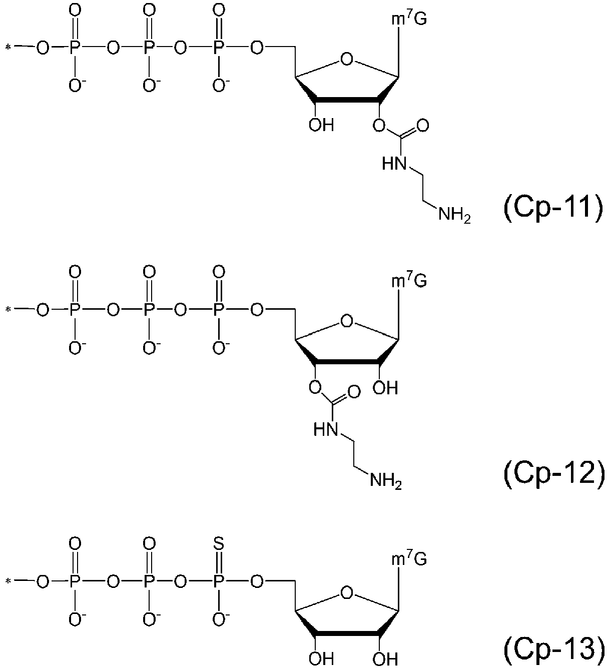 wherein m⁷G represents N7-methylguanine; and ^{∗} represents a bond that binds to a carbon atom at a 5'position of an adjacent nucleotide residue.

18. A modified RNA comprising a structure selected from the group consisting of the following formulas (Ca-1) to (Ca-3) at a 5' end, 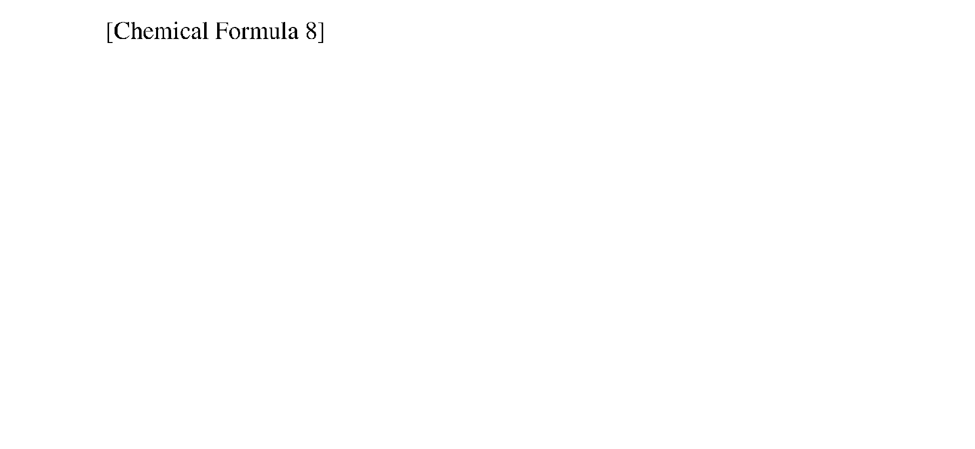 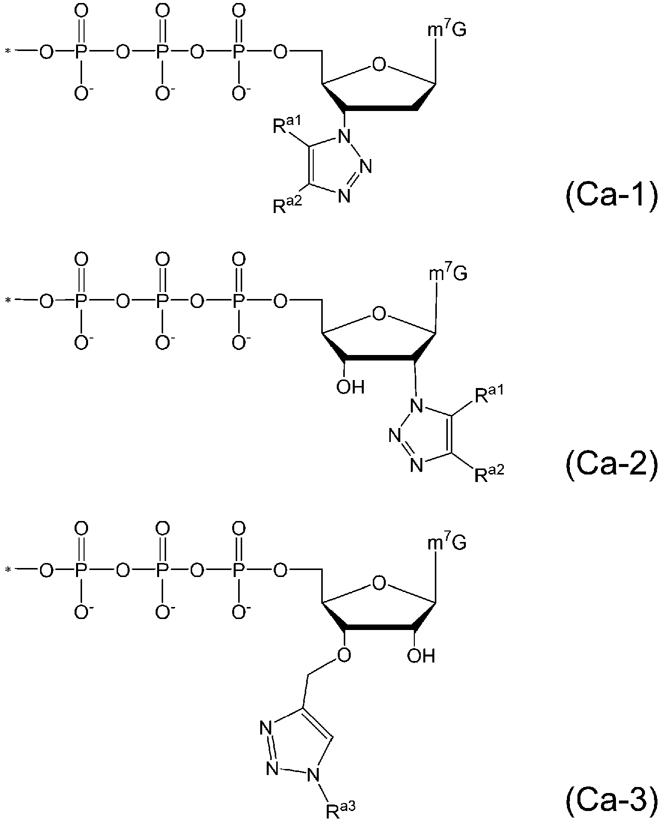
wherein m⁷G represents N7-methylguanine; and ^{∗} represents a bond that binds to a carbon atom at a 5'position of an adjacent nucleotide residue; R^{a1} and R^{a2} each independently represent a hydrogen atom or an organic group, provided that at least one of R^{a1} and R^{a2} is an organic group,
when both R^{a1} and R^{a2} are organic groups, they may bind with each other to form a ring structure, and
R^{a3} represents an organic group.

19. A modified circular RNA comprising a structure selected from the group consisting of the following formulas (Ci-1) to (Ci-3), 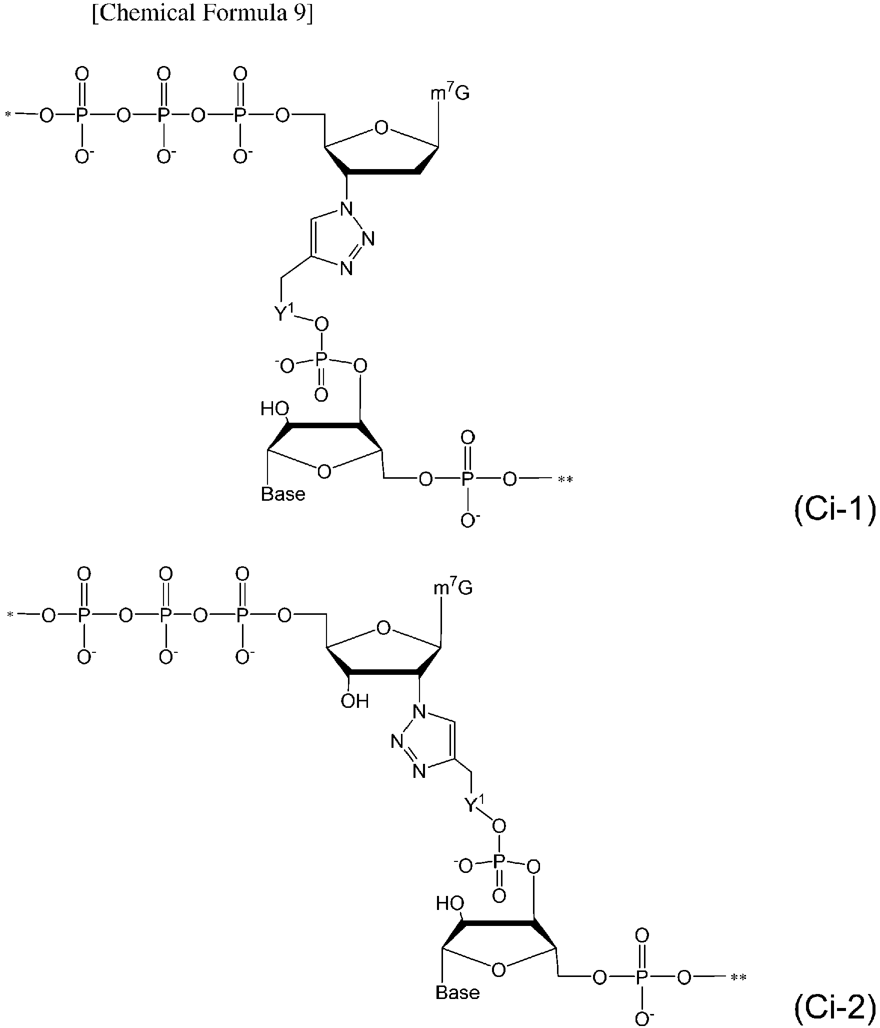 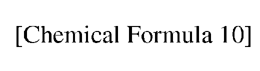 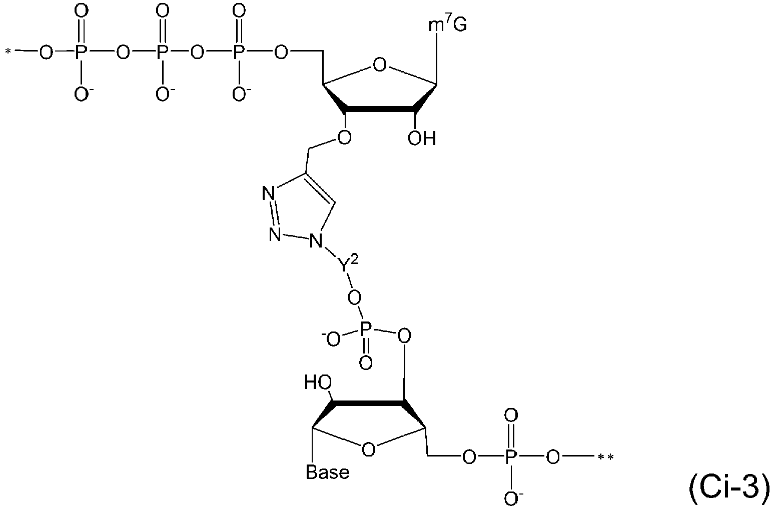 wherein m⁷G represents N7-methylguanine; Y¹ and Y² each independently represent a divalent linking group; Base each independently represents a nucleotide base, ^{∗} represents a bond that binds to a carbon atom at a 5'position of an adjacent nucleotide residue, and ^{∗∗} represents a bond that binds to a carbon atom at a 3'position of an adjacent nucleotide residue.


SEQUENCE LISTING

<110>  Kyoto University

<120>  Method of capping RNA, method of producing modified RNA, and
       modified RNA

<130>  PC-29654

<150>  JP19-073163
<151>  2019-04-05

<160>  5

<170>  PatentIn version 3.5

<210>  1
<211>  11
<212>  RNA
<213>  Artificial Sequence

<220>
<223>  short RNA for capping

<400>  1
gggcgaauua a                                                          11


<210>  2
<211>  2535
<212>  DNA
<213>  Vaccinia virus


<220>
<221>  CDS
<222>  (1)..(2535)

<400>  2
atg gat gcc aac gta gta tca tct tct act att gcg acg tat ata gac       48
Met Asp Ala Asn Val Val Ser Ser Ser Thr Ile Ala Thr Tyr Ile Asp
1               5                   10                  15

gct tta gcg aag aat gct tcg gaa tta gaa cag agg tct acc gca tac       96
Ala Leu Ala Lys Asn Ala Ser Glu Leu Glu Gln Arg Ser Thr Ala Tyr
            20                  25                  30

gaa ata aat aat gaa ttg gaa cta gta ttt att aag ccg cca ttg att      144
Glu Ile Asn Asn Glu Leu Glu Leu Val Phe Ile Lys Pro Pro Leu Ile
        35                  40                  45

act ttg aca aat gta gtg aat atc tct acg att cag gaa tcg ttt att      192
Thr Leu Thr Asn Val Val Asn Ile Ser Thr Ile Gln Glu Ser Phe Ile
    50                  55                  60

cga ttt acc gtt act aat aag gaa ggt gtt aaa att aga act aag att      240
Arg Phe Thr Val Thr Asn Lys Glu Gly Val Lys Ile Arg Thr Lys Ile
65                  70                  75                  80

cca tta tct aag gta cat ggt cta gat gta aaa aat gta cag tta gta      288
Pro Leu Ser Lys Val His Gly Leu Asp Val Lys Asn Val Gln Leu Val
                85                  90                  95

gat gct ata gat aac ata gtt tgg gaa aag aaa tca tta gtg acg gaa 336
Asp Ala Ile Asp Asn Ile Val Trp Glu Lys Lys Ser Leu Val Thr Glu
            100                 105                 110

aat cgt ctt cac aaa gaa tgc ttg ttg aga cta tcg aca gag gaa cgt 384
Asn Arg Leu His Lys Glu Cys Leu Leu Arg Leu Ser Thr Glu Glu Arg
        115                 120                 125

cat ata ttt ttg gat tac aag aaa tat gga tcc tct atc cga cta gaa 432
His Ile Phe Leu Asp Tyr Lys Lys Tyr Gly Ser Ser Ile Arg Leu Glu
    130                 135                 140

tta gtc aat ctt att caa gca aaa aca aaa aac ttt acg ata gac ttt 480
Leu Val Asn Leu Ile Gln Ala Lys Thr Lys Asn Phe Thr Ile Asp Phe
145                 150                 155                 160

aag cta aaa tat ttt cta gga tcc ggt gcc cag tct aaa agt tct tta 528
Lys Leu Lys Tyr Phe Leu Gly Ser Gly Ala Gln Ser Lys Ser Ser Leu
                165                 170                 175

tta cac gct att aat cat cca aag tca agg cct aat aca tct ctg gaa 576
Leu His Ala Ile Asn His Pro Lys Ser Arg Pro Asn Thr Ser Leu Glu
            180                 185                 190

ata gaa ttt aca cct aga gac aat gaa aca gtt cca tat gat gaa cta 624
Ile Glu Phe Thr Pro Arg Asp Asn Glu Thr Val Pro Tyr Asp Glu Leu
        195                 200                 205

ata aag gaa ttg acg act ctc tcg cgt cat ata ttt atg gct tct cca 672
Ile Lys Glu Leu Thr Thr Leu Ser Arg His Ile Phe Met Ala Ser Pro
    210                 215                 220

gag aat gta att ctt tct ccg cct att aac gcg cct ata aaa acc ttt 720
Glu Asn Val Ile Leu Ser Pro Pro Ile Asn Ala Pro Ile Lys Thr Phe
225                 230                 235                 240

atg ttg cct aaa caa gat ata gta ggt ttg gat ctg gaa aat cta tat 768
Met Leu Pro Lys Gln Asp Ile Val Gly Leu Asp Leu Glu Asn Leu Tyr
                245                 250                 255

gcc gta act aag act gac ggc att cct ata act atc aga gtt aca tca 816
Ala Val Thr Lys Thr Asp Gly Ile Pro Ile Thr Ile Arg Val Thr Ser
            260                 265                 270

aac ggg ttg tat tgt tat ttt aca cat ctt ggt tat att att aga tat 864
Asn Gly Leu Tyr Cys Tyr Phe Thr His Leu Gly Tyr Ile Ile Arg Tyr
        275                 280                 285

cct gtt aag aga ata ata gat tcc gaa gta gta gtc ttt ggt gag gca 912
Pro Val Lys Arg Ile Ile Asp Ser Glu Val Val Val Phe Gly Glu Ala
    290                 295                 300

gtt aag gat aag aac tgg acc gta tat ctc att aag cta ata gag cct 960
Val Lys Asp Lys Asn Trp Thr Val Tyr Leu Ile Lys Leu Ile Glu Pro
305                 310                 315                 320

gtg aat gca atc aat gat aga cta gaa gaa agt aag tat gtt gaa tct 1008
Val Asn Ala Ile Asn Asp Arg Leu Glu Glu Ser Lys Tyr Val Glu Ser
                325                 330                 335

aaa cta gtg gat att tgt gat cgg ata gta ttc aag tca aag aaa tac 1056
Lys Leu Val Asp Ile Cys Asp Arg Ile Val Phe Lys Ser Lys Lys Tyr
            340                 345                 350

gaa ggt ccg ttt act aca act agt gaa gtc gtc gat atg tta tct aca 1104
Glu Gly Pro Phe Thr Thr Thr Ser Glu Val Val Asp Met Leu Ser Thr
        355                 360                 365

tat tta cca aag caa cca gaa ggt gtt att ctg ttc tat tca aag gga 1152
Tyr Leu Pro Lys Gln Pro Glu Gly Val Ile Leu Phe Tyr Ser Lys Gly
    370                 375                 380

cct aaa tct aac att gat ttt aaa att aaa aag gaa aat act ata gac 1200
Pro Lys Ser Asn Ile Asp Phe Lys Ile Lys Lys Glu Asn Thr Ile Asp
385                 390                 395                 400

caa act gca aat gta gta ttt agg tac atg tcc agt gaa cca att atc 1248
Gln Thr Ala Asn Val Val Phe Arg Tyr Met Ser Ser Glu Pro Ile Ile
                405                 410                 415

ttt gga gag tcg tct atc ttt gta gag tat aag aaa ttt agc aac gat 1296
Phe Gly Glu Ser Ser Ile Phe Val Glu Tyr Lys Lys Phe Ser Asn Asp
            420                 425                 430

aaa ggc ttt cct aaa gaa tat ggt tct ggt aag att gtg tta tat aac 1344
Lys Gly Phe Pro Lys Glu Tyr Gly Ser Gly Lys Ile Val Leu Tyr Asn
        435                 440                 445

ggc gtt aat tat cta aat aat atc tat tgt ttg gaa tat att aat aca 1392
Gly Val Asn Tyr Leu Asn Asn Ile Tyr Cys Leu Glu Tyr Ile Asn Thr
    450                 455                 460

cat aat gaa gtg ggt att aag tcc gtg gtt gta cct att aag ttt ata 1440
His Asn Glu Val Gly Ile Lys Ser Val Val Val Pro Ile Lys Phe Ile
465                 470                 475                 480

gca gaa ttc tta gtt aat gga gaa ata ctt aaa cct aga att gat aaa 1488
Ala Glu Phe Leu Val Asn Gly Glu Ile Leu Lys Pro Arg Ile Asp Lys
                485                 490                 495

acc atg aaa tat att aac tca gaa gat tat tat gga aat caa cat aat 1536
Thr Met Lys Tyr Ile Asn Ser Glu Asp Tyr Tyr Gly Asn Gln His Asn
            500                 505                 510

atc ata gtc gaa cat tta aga gat caa agc atc aaa ata gga gat atc 1584
Ile Ile Val Glu His Leu Arg Asp Gln Ser Ile Lys Ile Gly Asp Ile
        515                 520                 525

ttt aac gag gat aaa cta tcg gat gtg gga cat caa tac gcc aat aat 1632
Phe Asn Glu Asp Lys Leu Ser Asp Val Gly His Gln Tyr Ala Asn Asn
    530                 535                 540

gat aaa ttt aga tta aat cca gaa gtt agt tat ttt acg aat aaa cga 1680
Asp Lys Phe Arg Leu Asn Pro Glu Val Ser Tyr Phe Thr Asn Lys Arg
545                 550                 555                 560

act aga gga ccg ttg gga att tta tca aac tac gtc aag act ctt ctt 1728
Thr Arg Gly Pro Leu Gly Ile Leu Ser Asn Tyr Val Lys Thr Leu Leu
                565                 570                 575

att tct atg tat tgt tcc aaa aca ttt tta gac gat tcc aac aaa cga 1776
Ile Ser Met Tyr Cys Ser Lys Thr Phe Leu Asp Asp Ser Asn Lys Arg
            580                 585                 590

aag gta ttg gcg att gat ttt gga aac ggt gct gac ctg gaa aaa tac 1824
Lys Val Leu Ala Ile Asp Phe Gly Asn Gly Ala Asp Leu Glu Lys Tyr

595                 600                 605

ttt tat gga gag att gcg tta ttg gta gcg acg gat ccg gat gct gat        1872
Phe Tyr Gly Glu Ile Ala Leu Leu Val Ala Thr Asp Pro Asp Ala Asp
    610                 615                 620

gct ata gct aga gga aat gaa aga tac aac aaa tta aac tct gga att        1920
Ala Ile Ala Arg Gly Asn Glu Arg Tyr Asn Lys Leu Asn Ser Gly Ile
625                 630                 635                 640

aaa acc aag tac tac aaa ttt gac tac att cag gaa act att cga tcc        1968
Lys Thr Lys Tyr Tyr Lys Phe Asp Tyr Ile Gln Glu Thr Ile Arg Ser
                645                 650                 655

gat aca ttt gtc tct agt gtc aga gaa gta ttc tat ttt gga aag ttt        2016
Asp Thr Phe Val Ser Ser Val Arg Glu Val Phe Tyr Phe Gly Lys Phe
            660                 665                 670

aat atc atc gac tgg cag ttt gct atc cat tat tct ttt cat ccg aga        2064
Asn Ile Ile Asp Trp Gln Phe Ala Ile His Tyr Ser Phe His Pro Arg
        675                 680                 685

cat tat gct acc gtc atg aat aac tta tcc gaa cta act gct tct gga        2112
His Tyr Ala Thr Val Met Asn Asn Leu Ser Glu Leu Thr Ala Ser Gly
    690                 695                 700

ggc aag gta tta atc act acc atg gac gga gac aaa tta tca aaa tta        2160
Gly Lys Val Leu Ile Thr Thr Met Asp Gly Asp Lys Leu Ser Lys Leu
705                 710                 715                 720

aca gat aaa aag act ttt ata att cat aag aat tta cct agt agc gaa        2208
Thr Asp Lys Lys Thr Phe Ile Ile His Lys Asn Leu Pro Ser Ser Glu
                725                 730                 735

aac tat atg tct gta gaa aaa ata gct gat gat aga ata gtg gta tat        2256
Asn Tyr Met Ser Val Glu Lys Ile Ala Asp Asp Arg Ile Val Val Tyr
            740                 745                 750

aat cca tca aca atg tct act cca atg act gaa tac att atc aaa aag        2304
Asn Pro Ser Thr Met Ser Thr Pro Met Thr Glu Tyr Ile Ile Lys Lys
        755                 760                 765

aac gat ata gtc aga gtg ttt aac gaa tac gga ttt gtt ctt gta gat        2352
Asn Asp Ile Val Arg Val Phe Asn Glu Tyr Gly Phe Val Leu Val Asp
    770                 775                 780

aac gtt gat ttc gct aca att ata gaa cga agt aaa aag ttt att aat        2400
Asn Val Asp Phe Ala Thr Ile Ile Glu Arg Ser Lys Lys Phe Ile Asn
785                 790                 795                 800

ggc gca tct aca atg gaa gat aga cca tct aca aga aac ttt ttc gaa        2448
Gly Ala Ser Thr Met Glu Asp Arg Pro Ser Thr Arg Asn Phe Phe Glu
                805                 810                 815

cta aat aga gga gcc att aaa tgt gaa ggt tta gat gtc gaa gac tta        2496
Leu Asn Arg Gly Ala Ile Lys Cys Glu Gly Leu Asp Val Glu Asp Leu
            820                 825                 830

ctt agt tac tat gtt gtt tat gtc ttt tct aag cgg taa                    2535
Leu Ser Tyr Tyr Val Val Tyr Val Phe Ser Lys Arg
        835                 840

<210>  3
<211>  844
<212>  PRT
<213>  Vaccinia virus

<400>  3

Met Asp Ala Asn Val Val Ser Ser Ser Thr Ile Ala Thr Tyr Ile Asp
1               5                   10                  15

Ala Leu Ala Lys Asn Ala Ser Glu Leu Glu Gln Arg Ser Thr Ala Tyr
            20                  25                  30

Glu Ile Asn Asn Glu Leu Glu Leu Val Phe Ile Lys Pro Pro Leu Ile
        35                  40                  45

Thr Leu Thr Asn Val Val Asn Ile Ser Thr Ile Gln Glu Ser Phe Ile
    50                  55                  60

Arg Phe Thr Val Thr Asn Lys Glu Gly Val Lys Ile Arg Thr Lys Ile
65                  70                  75                  80

Pro Leu Ser Lys Val His Gly Leu Asp Val Lys Asn Val Gln Leu Val
                85                  90                  95

Asp Ala Ile Asp Asn Ile Val Trp Glu Lys Lys Ser Leu Val Thr Glu
            100                 105                 110

Asn Arg Leu His Lys Glu Cys Leu Leu Arg Leu Ser Thr Glu Glu Arg
        115                 120                 125

His Ile Phe Leu Asp Tyr Lys Lys Tyr Gly Ser Ser Ile Arg Leu Glu
    130                 135                 140

Leu Val Asn Leu Ile Gln Ala Lys Thr Lys Asn Phe Thr Ile Asp Phe
145                 150                 155                 160

Lys Leu Lys Tyr Phe Leu Gly Ser Gly Ala Gln Ser Lys Ser Ser Leu
                165                 170                 175

Leu His Ala Ile Asn His Pro Lys Ser Arg Pro Asn Thr Ser Leu Glu
            180                 185                 190

Ile Glu Phe Thr Pro Arg Asp Asn Glu Thr Val Pro Tyr Asp Glu Leu
        195                 200                 205

Ile Lys Glu Leu Thr Thr Leu Ser Arg His Ile Phe Met Ala Ser Pro
    210                 215                 220

Glu Asn Val Ile Leu Ser Pro Pro Ile Asn Ala Pro Ile Lys Thr Phe
225                 230                 235                 240

Met Leu Pro Lys Gln Asp Ile Val Gly Leu Asp Leu Glu Asn Leu Tyr
                245                 250                 255

Ala Val Thr Lys Thr Asp Gly Ile Pro Ile Thr Ile Arg Val Thr Ser
            260                 265                 270

Asn Gly Leu Tyr Cys Tyr Phe Thr His Leu Gly Tyr Ile Ile Arg Tyr
        275                 280                 285

Pro Val Lys Arg Ile Ile Asp Ser Glu Val Val Val Phe Gly Glu Ala
    290                 295                 300

Val Lys Asp Lys Asn Trp Thr Val Tyr Leu Ile Lys Leu Ile Glu Pro
305                 310                 315                 320

Val Asn Ala Ile Asn Asp Arg Leu Glu Glu Ser Lys Tyr Val Glu Ser
                325                 330                 335

Lys Leu Val Asp Ile Cys Asp Arg Ile Val Phe Lys Ser Lys Lys Tyr
            340                 345                 350

Glu Gly Pro Phe Thr Thr Thr Ser Glu Val Val Asp Met Leu Ser Thr
        355                 360                 365

Tyr Leu Pro Lys Gln Pro Glu Gly Val Ile Leu Phe Tyr Ser Lys Gly
    370                 375                 380

Pro Lys Ser Asn Ile Asp Phe Lys Ile Lys Lys Glu Asn Thr Ile Asp
385                 390                 395                 400

Gln Thr Ala Asn Val Val Phe Arg Tyr Met Ser Ser Glu Pro Ile Ile
                405                 410                 415

Phe Gly Glu Ser Ser Ile Phe Val Glu Tyr Lys Lys Phe Ser Asn Asp
            420                 425                 430

Lys Gly Phe Pro Lys Glu Tyr Gly Ser Gly Lys Ile Val Leu Tyr Asn
        435                 440                 445

Gly Val Asn Tyr Leu Asn Asn Ile Tyr Cys Leu Glu Tyr Ile Asn Thr
    450                 455                 460

His Asn Glu Val Gly Ile Lys Ser Val Val Val Pro Ile Lys Phe Ile
465                 470                 475                 480

Ala Glu Phe Leu Val Asn Gly Glu Ile Leu Lys Pro Arg Ile Asp Lys
                485                 490                 495

Thr Met Lys Tyr Ile Asn Ser Glu Asp Tyr Tyr Gly Asn Gln His Asn
            500                 505                 510

Ile Ile Val Glu His Leu Arg Asp Gln Ser Ile Lys Ile Gly Asp Ile
        515                 520                 525

Phe Asn Glu Asp Lys Leu Ser Asp Val Gly His Gln Tyr Ala Asn Asn
    530                 535                 540

Asp Lys Phe Arg Leu Asn Pro Glu Val Ser Tyr Phe Thr Asn Lys Arg
545                 550                 555                 560

Thr Arg Gly Pro Leu Gly Ile Leu Ser Asn Tyr Val Lys Thr Leu Leu
                565                 570                 575

Ile Ser Met Tyr Cys Ser Lys Thr Phe Leu Asp Asp Ser Asn Lys Arg
            580                 585                 590

Lys Val Leu Ala Ile Asp Phe Gly Asn Gly Ala Asp Leu Glu Lys Tyr
        595                 600                 605

Phe Tyr Gly Glu Ile Ala Leu Leu Val Ala Thr Asp Pro Asp Ala Asp
    610                 615                 620

Ala Ile Ala Arg Gly Asn Glu Arg Tyr Asn Lys Leu Asn Ser Gly Ile
625                 630                 635                 640

Lys Thr Lys Tyr Tyr Lys Phe Asp Tyr Ile Gln Glu Thr Ile Arg Ser
                645                 650                 655

Asp Thr Phe Val Ser Ser Val Arg Glu Val Phe Tyr Phe Gly Lys Phe
            660                 665                 670

Asn Ile Ile Asp Trp Gln Phe Ala Ile His Tyr Ser Phe His Pro Arg
        675                 680                 685

His Tyr Ala Thr Val Met Asn Asn Leu Ser Glu Leu Thr Ala Ser Gly
    690                 695                 700

Gly Lys Val Leu Ile Thr Thr Met Asp Gly Asp Lys Leu Ser Lys Leu
705                 710                 715                 720

Thr Asp Lys Lys Thr Phe Ile Ile His Lys Asn Leu Pro Ser Ser Glu
                725                 730                 735

Asn Tyr Met Ser Val Glu Lys Ile Ala Asp Asp Arg Ile Val Val Tyr
            740                 745                 750

Asn Pro Ser Thr Met Ser Thr Pro Met Thr Glu Tyr Ile Ile Lys Lys
        755                 760                 765

Asn Asp Ile Val Arg Val Phe Asn Glu Tyr Gly Phe Val Leu Val Asp
    770                 775                 780

Asn Val Asp Phe Ala Thr Ile Ile Glu Arg Ser Lys Lys Phe Ile Asn
785                 790                 795                 800

Gly Ala Ser Thr Met Glu Asp Arg Pro Ser Thr Arg Asn Phe Phe Glu
                805                 810                 815

Leu Asn Arg Gly Ala Ile Lys Cys Glu Gly Leu Asp Val Glu Asp Leu
            820                 825                 830

Leu Ser Tyr Tyr Val Val Tyr Val Phe Ser Lys Arg
        835                 840

<210> 4
<211> 864
<212> DNA
<213> Vaccinia virus

<220>
<221> CDS
<222> (1)..(864)

<400> 4
atg gat gaa att gta aaa aat atc cgg gag gga acg cat gtc ctt ctt 48
Met Asp Glu Ile Val Lys Asn Ile Arg Glu Gly Thr His Val Leu Leu
1               5                   10                  15

cca ttt tat gaa aca ttg cca gaa ctt aat ctg tct cta ggt aaa agc 96
Pro Phe Tyr Glu Thr Leu Pro Glu Leu Asn Leu Ser Leu Gly Lys Ser
            20                  25                  30

cca tta cct agt ctg gaa tac gga gct aat tac ttt ctt cag att tct 144
Pro Leu Pro Ser Leu Glu Tyr Gly Ala Asn Tyr Phe Leu Gln Ile Ser
        35                  40                  45

aga gtt aat gat cta aat aga atg ccg acc gac atg tta aaa ctt ttt 192
Arg Val Asn Asp Leu Asn Arg Met Pro Thr Asp Met Leu Lys Leu Phe
    50                  55                  60

aca cat gat atc atg tta cca gaa agc gat cta gat aaa gtc tat gaa 240
Thr His Asp Ile Met Leu Pro Glu Ser Asp Leu Asp Lys Val Tyr Glu
65                  70                  75                  80

att tta aag att aat agc gta aag tat tat ggg agg agt act aaa gcg 288
Ile Leu Lys Ile Asn Ser Val Lys Tyr Tyr Gly Arg Ser Thr Lys Ala

85                  90                  95

gac gcc gta gtt gcc gac ctc agc gca cgc aat aaa ctg ttc aaa cgt        336
Asp Ala Val Val Ala Asp Leu Ser Ala Arg Asn Lys Leu Phe Lys Arg
            100                 105                 110

gaa cga gat gct att aaa tct aat aat cat ctc act gaa aac aat cta        384
Glu Arg Asp Ala Ile Lys Ser Asn Asn His Leu Thr Glu Asn Asn Leu
        115                 120                 125

tac att agc gat tat aag atg tta acc ttc gac gtg ttt cga cca tta        432
Tyr Ile Ser Asp Tyr Lys Met Leu Thr Phe Asp Val Phe Arg Pro Leu
    130                 135                 140

ttt gat ttt gta aac gaa aaa tat tgt att att aaa ctt cca act tta        480
Phe Asp Phe Val Asn Glu Lys Tyr Cys Ile Ile Lys Leu Pro Thr Leu
145                 150                 155                 160

ttc ggt aga ggt gta atc gat act atg aga ata tat tgt agt ctc ttt        528
Phe Gly Arg Gly Val Ile Asp Thr Met Arg Ile Tyr Cys Ser Leu Phe
                165                 170                 175

aaa aat gtt aga ctg cta aaa tgc gta agc gat agc tgg tta aaa gat        576
Lys Asn Val Arg Leu Leu Lys Cys Val Ser Asp Ser Trp Leu Lys Asp
            180                 185                 190

agc gcc att atg gtg gct agt gat gtt tgt aaa aaa aat ttg gat tta        624
Ser Ala Ile Met Val Ala Ser Asp Val Cys Lys Lys Asn Leu Asp Leu
        195                 200                 205

ttt atg tct cat gtt aag tcc gtc act aag tct tct tct tgg aag gat        672
Phe Met Ser His Val Lys Ser Val Thr Lys Ser Ser Ser Trp Lys Asp
    210                 215                 220

gtg aac agt gtt caa ttt agt att tta aac aat cca gtg gat acg gaa        720
Val Asn Ser Val Gln Phe Ser Ile Leu Asn Asn Pro Val Asp Thr Glu
225                 230                 235                 240

ttc att aat aag ttc tta gag ttt tcg aat aga gta tac gaa gct ctc        768
Phe Ile Asn Lys Phe Leu Glu Phe Ser Asn Arg Val Tyr Glu Ala Leu
                245                 250                 255

tat tac gtt cac tcg ttg ctt tat tct agt atg act tct gat tca aaa        816
Tyr Tyr Val His Ser Leu Leu Tyr Ser Ser Met Thr Ser Asp Ser Lys
            260                 265                 270

agt atc gaa aac aaa cat cag aga aga cta gtt aaa cta ctg ctg tga        864
Ser Ile Glu Asn Lys His Gln Arg Arg Leu Val Lys Leu Leu Leu
        275                 280                 285

<210>  5
<211>  287
<212>  PRT
<213>  Vaccinia virus

<400>  5

Met Asp Glu Ile Val Lys Asn Ile Arg Glu Gly Thr His Val Leu Leu
1               5                   10                  15

Pro Phe Tyr Glu Thr Leu Pro Glu Leu Asn Leu Ser Leu Gly Lys Ser

            20                  25                  30

Pro Leu Pro Ser Leu Glu Tyr Gly Ala Asn Tyr Phe Leu Gln Ile Ser
        35                  40                  45

Arg Val Asn Asp Leu Asn Arg Met Pro Thr Asp Met Leu Lys Leu Phe
    50                  55                  60

Thr His Asp Ile Met Leu Pro Glu Ser Asp Leu Asp Lys Val Tyr Glu
65                  70                  75                  80

Ile Leu Lys Ile Asn Ser Val Lys Tyr Tyr Gly Arg Ser Thr Lys Ala
                85                  90                  95

Asp Ala Val Val Ala Asp Leu Ser Ala Arg Asn Lys Leu Phe Lys Arg
            100                 105                 110

Glu Arg Asp Ala Ile Lys Ser Asn Asn His Leu Thr Glu Asn Asn Leu
        115                 120                 125

Tyr Ile Ser Asp Tyr Lys Met Leu Thr Phe Asp Val Phe Arg Pro Leu
    130                 135                 140

Phe Asp Phe Val Asn Glu Lys Tyr Cys Ile Ile Lys Leu Pro Thr Leu
145                 150                 155                 160

Phe Gly Arg Gly Val Ile Asp Thr Met Arg Ile Tyr Cys Ser Leu Phe
                165                 170                 175

Lys Asn Val Arg Leu Leu Lys Cys Val Ser Asp Ser Trp Leu Lys Asp
            180                 185                 190

Ser Ala Ile Met Val Ala Ser Asp Val Cys Lys Lys Asn Leu Asp Leu
        195                 200                 205

Phe Met Ser His Val Lys Ser Val Thr Lys Ser Ser Ser Trp Lys Asp
    210                 215                 220

Val Asn Ser Val Gln Phe Ser Ile Leu Asn Asn Pro Val Asp Thr Glu
225                 230                 235                 240

Phe Ile Asn Lys Phe Leu Glu Phe Ser Asn Arg Val Tyr Glu Ala Leu
                245                 250                 255

Tyr Tyr Val His Ser Leu Leu Tyr Ser Ser Met Thr Ser Asp Ser Lys
            260                 265                 270

Ser Ile Glu Asn Lys His Gln Arg Arg Leu Val Lys Leu Leu Leu
        275                 280                 285